# EUROPEAN PATENT SPECIFICATION

(11) **EP 4 136 207 B1**
(45) Date of publication and mention of the grant of the patent: **24.04.2024**
(21) Application number: 21734009.0
(22) Date of filing: 23.06.2021
(51) Int. Cl.: C12M 3/06, C12M 1/00, C12N 5/00, C12N 5/076, C12M 1/34, C12M 1/36

(54) **SAMPLE SEPARATING METHOD**
VERFAHREN ZUR PROBENTRENNUNG
SYSTÈME DE SÉPARATION D'ÉCHANTILLONS

(30) Priority: 24.06.2020 EP 20182102; 20.04.2021 EP 21169511
(43) Date of publication of application: 22.02.2023
(73) Proprietor: University of Limerick, Limerick V94 T9PX (IE)
(72) Inventor: FAIR, Sean, Limerick, V94 T9PX (IE); NEWPORT, David, Limerick, V94 T9PX (IE); ROMERO AGUIRREGOMEZCORTA, Jon, Limerick, V94 T9PX (IE); O'SULLIVAN, Leonard, Limerick, V94 T9PX (IE); WHITE, Eoin, Limerick, V94 T9PX (IE); O'SULLIVAN, Kevin, Limerick, V94 T9PX (IE)
(74) Representative: Appleyard Lees IP LLP
(86) International application number: PCT/EP2021/067161
(87) International publication number: WO 2021/260012

(56) References cited:
- WO-A1-2016/035799
- WO-A1-2020/041303
- DUCK-BONG SEO ET AL: "Development of sorting, aligning, and orienting motile sperm using microfluidic device operated by hydrostatic pressure", MICROFLUIDICS AND NANOFLUIDICS, SPRINGER, BERLIN, DE, vol. 3, no. 5, 10 January 2007 (2007-01-10), pages 561-570, XP019522724, ISSN: 1613-4990, DOI: 10.1007/S10404-006-0142-3

## Description

The present invention is directed towards a sample separating method, and in particular a sample separating method for separating motile organisms from other organisms.

The present invention is directed towards a sample separating device, and in particular a sample separating device for separating motile organisms from other organisms.

The present invention is directed towards a sample separating apparatus, and in particular a sample separating apparatus for separating motile organisms from other organisms.

The sample separating device on which the method of the present invention is applied may be for separating motile sperm from an original sperm sample comprising a mixture of motile sperm, dead sperm, deformed sperm and other non-motile organisms. It may be desirable to separate the motile sperm from the other organisms in the sample as part of a preparation purpose for preparing sperm for an assisted fertilisation procedure.

An example of an assisted fertilisation procedure is artificial insemination which is also known as intrauterine insemination. Artificial insemination is a common procedure used in the field of livestock reproduction, and is also used as an infertility treatment for humans. Artificial insemination involves the introduction of sperm into a female's cervix or uterine cavity by means other than sexual intercourse.

Another example of an assisted fertilisation procedure is in vitro fertilisation (IVF). IVF refers to a fertilisation procedure where an oocyte is combined with sperm outside of the body. In one example of this, a large number of sperm may be placed together with the oocyte and incubated so that fertilisation may take place. In another example of this, a single sperm may be injected directly into the oocyte in a process known as intra cytoplasmic sperm injection (ICSI). ICSI may be used, in particular, when the sperm donor has a low sperm count or low average sperm motility.

In all of the above assisted fertilisation procedures, it is desirable to separate the motile sperm from the other organisms within the sample. This is so as to increase the chances of the fertilisation procedure being successful. This is particularly the case for ICSI, where an embryologist is required to select one sperm for injection into the oocyte. Providing a high quality motile sperm sample with minimal sperm having abnormalities will make the selection process easier for the embryologist, and will reduce the failure rate of the ICSI procedure.

Known approaches to separating motile sperm from other organisms exploit the fact that motile sperm swim against a fluid flow. This property of sperm, and other organisms, to swim against a fluid flow (a current) is known as rheotaxis.

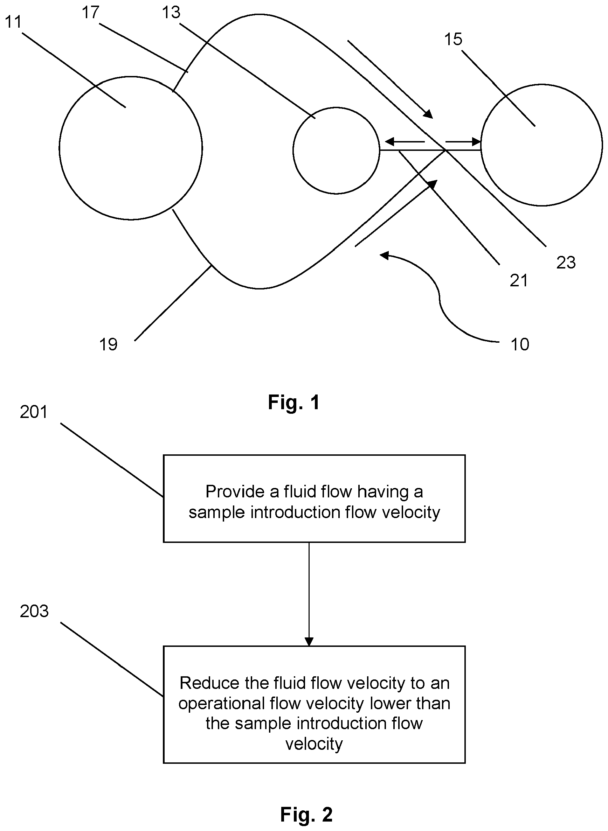

Referring to Figure 1, there is shown an example of a known microfluidic sperm sorting device 10. The sperm sorting device 10 comprises a fluid reservoir 11, a sperm introduction zone in the form of a chamber 13, and a sperm collection zone in the form of a chamber 15. A channel arrangement is provided that includes a primary fluid path 17, 19 between the fluid reservoir 11 and the collection chamber 15. The channel arrangement includes a secondary fluid path 21 between the sperm chamber 13 and a junction point 23.

In use, the fluid reservoir 11 is filled with a fluid, and a sperm sample is introduced into the sperm chamber 13. The relative height difference between the fluid in the fluid reservoir 11 and the sperm chamber 13/collection chamber 15 causes fluid to flow through the primary fluid path 17, 19 towards the collection chamber 15,and through the secondary fluid path 21 into the sperm chamber 13. Motile sperm are able to swim against the fluid flow by rheotaxis, exit the sperm chamber 13, and reach the junction 23. The motile sperm are then swept up in the fluid flow of the primary fluid path 17, 19 and enter the collection chamber 15. The non-motile sperm are unable to move against the fluid flow and thus remain in the sperm chamber 13. During the sperm sorting operation, the fluid level in the fluid reservoir 11 decreases until there is no relative height difference between the fluid reservoir 11, sperm chamber 13, and collection chamber 15. The intention behind the device of Figure 1 is that only motile sperm enter the collection chamber 15.

An example of the prior art is shown in WO2020041303, which discloses systems and methods for sperm selection.

It is desirable to improve on existing approaches for separating motile organisms from other organisms, such as by increasing the relative proportion of motile organisms in the sample collection zone, and by reducing the relative proportion of other organisms in the sample collection zone.

It is an object of the present invention to improve on, or at least provide an alternative to, existing approaches for separating motile organisms form other organisms.

According to the present invention there is provided an apparatus and method as set forth in the appended claims. Other features of the invention will be apparent from the dependent claims, and the description which follows.

According to a first aspect of the invention, there is provided a sample separating method for separating motile organisms from other organisms. The method comprises controlling a fluid delivery arrangement to provide a fluid flow to a sample separating device comprising a fluid inlet and a fluid terminus. The fluid flow is provided between the fluid inlet and the fluid terminus of the device. The fluid flow has a sample introduction flow velocity set so that a sample may be introduced into a sample introduction zone of the device. The sample introduction flow velocity is sufficiently high such that an organism in the sample is unable to exit the sample introduction zone. The method comprises controlling the fluid delivery arrangement to reduce the fluid flow velocity from the sample introduction flow velocity to an operational flow velocity lower than the sample introduction flow velocity. The operational flow velocity is selected such that motile organisms in the sample are able to swim against the fluid flow and enter a sample collection zone of the device.

Here and throughout, "fluid inlet" may be understood as a part of the sample separating device where a fluid may be introduced. Here and throughout, "fluid terminus" may be understood as the end point of the fluid flow in the sample separating device. The fluid terminus may be in the form of a well to collect fluid. The fluid terminus may be in the form of a fluid outlet which allows fluid to flow out of the device.

Here and throughout, "flow velocity" may be understood as meaning the average velocity. The average velocity may refer to the average velocity across a cross sectional area. The cross-sectional area may refer to an area of a channel between the fluid inlet and the fluid terminus. That is, the flow velocity may mean the flow velocity per channel. In particular, the flow velocity may mean the flow velocity through the channel where the selection process occurs. The channel may be between the sample collection zone and the sample introduction zone. In other words, the cross sectional area may refer to the area of a region where motile organisms (such as sperm) travel against the flow from the sample introduction zone to the sample collection zone. The method is particularly advantageous when used in conjunction with channels of constant cross sectional area.

Significantly, the present invention sets the fluid flow to have a sample introduction flow velocity that is sufficiently high so as to prevent organisms escaping the sample introduction zone of the device. In existing approaches, where a fluid flow is not provided when the sample is introduced into the sample introduction zone, or where the fluid flow has a low velocity, motile organisms and non-motile organisms may be able to escape the sample introduction zone as or shortly after they are introduced into the sample introduction zone. One reason for this is that organisms may travel up a channel between the sample introduction zone and the sample collection zone due to processes such as capillary action. Another reason for this is that the sample may be pipetted into the sample introduction zone, and the force with which they are pipetted may cause the sample to escape the sample introduction zone and travel towards the sample collection zone. The use of the sample introduction flow velocity is able to prevent the unwanted escape of organisms, such as non-motile or dead organisms, into the sample collection zone.

Moreover, the method further comprises reducing the fluid flow velocity from the sample introduction flow velocity to an operational flow velocity lower than the sample introduction velocity. This reduction in the flow velocity may be performed once the sample has been introduced into the sample introduction zone. The operational flow velocity is selected such that motile organisms are able to swim against the fluid flow and enter the sample collection zone. In this way, the present invention is able to control the fluid flow velocity so as to prevent organisms other than the desired motile organisms for entering the sample collection zone.

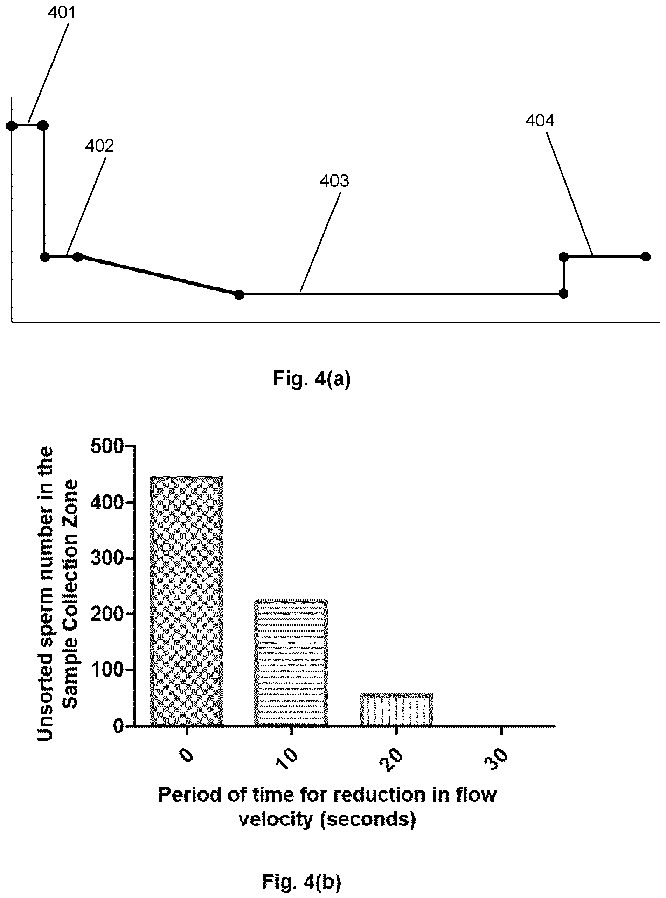

Controlling the fluid delivery arrangement to set the fluid flow to have the operational flow velocity comprises reducing the fluid flow velocity from the sample introduction flow velocity to the operational flow velocity over a predetermined period of time. This means that the fluid flow velocity is reduced gradually or incrementally from the sample introduction flow velocity to the operational flow velocity. This is highly advantageous, as it ensures that non-motile sperm do not reach the sample collection zone to contaminate the sample. This is contrasted with an "abrupt", "instantaneous", or "sudden" reduction from the sample introduction flow velocity to the operational flow velocity, which has been found to be detrimental and disadvantageous as it results in non-motile sperm being moved to the sample collection zone. An example of this is provided and explained with reference to Figure 4(b). That is, reduction of the fluid flow velocity from the sample introduction flow velocity to the operational flow velocity over a predetermined period of time is not an abrupt, sudden or instantaneous reduction. Instead, the reduction may be described as gradual or incremental as the reduction takes place over said predetermined time period. That is, the reduction in flow velocity is a gradual reduction of velocity, which is advantageous over an abrupt change of velocity.

The predetermined period of time may be greater than or equal to 1 minutes. Preferably, greater than or equal to 5 minutes. Preferably still, greater than or equal to 10 minutes.

The predetermined period of time may be between 2 minutes and 60 minutes. The predetermined period of time may be between 5 minutes and 60 minutes. The predetermined period of time may be between 10 minutes and 60 minutes. The predetermined period of time may be between 20 minutes and 60 minutes. The predetermined period of time may be between 30 minutes and 60 minutes. The predetermined period of time may be between 40 and 60 minutes. The predetermined period of time may be between 50 minutes and 60 minutes.

The predetermined period of time may be between 2 minutes and 50 minutes. The predetermined period of time may be between 2 minutes and 40 minutes. The predetermined period of time may be between 2 minutes and 30 minutes. The predetermined period of time may be between 2 minutes and 20 minutes. The predetermined period of time may be between 2 minutes and 10 minutes. The predetermined period of time may be between 2 minutes and 5 minutes.

Most preferably, the predetermined period of time may be greater than or equal to 1 minute. In a particular, preferred, example, the predetermined period of time may be between 1 minute and 60 minutes. In a particular, preferred example, the predetermined period of time may be between 1 minute and 20 minutes. In a highly preferred example, the predetermined period of time may be 1 minute.

In one highly preferred example, the predetermined period of time may be greater than or equal to 30 seconds. That is, the flow velocity is reduced over this predetermined period of time, which is a gradual (i.e. non abrupt) reduction in the flow velocity. This has been found to be highly beneficial in preventing organisms other than the desired motile organisms for entering the sample collection zone.

The sample introduction flow velocity may be greater than or equal to 100 micrometres per second. Preferably, the sample introduction flow velocity may be greater than or equal to 120 micrometres per second. In a particular, preferred, example, the sample introduction flow velocity may be 550 micrometres per second.

The sample introduction flow velocity may be between 100 micrometres per second to 1000 micrometres per second. The sample introduction flow velocity may be between 200 micrometres per second to 1000 micrometres per second. The sample introduction flow velocity may be between 200 micrometres per second to 800 micrometres per second. The sample introduction flow velocity may be between 300 micrometres per second to 1000 micrometres per second. The sample introduction flow velocity may be between 400 micrometres per second to 1000 micrometres per second. The sample introduction flow velocity may be between 500 micrometres per second to 1000 micrometres per second. The sample introduction flow velocity may be between 600 micrometres per second to 1000 micrometres per second. The sample introduction flow velocity may be between 700 micrometres per second to 1000 micrometres per second. The sample introduction flow velocity may be between 800 micrometres per second to 1000 micrometres per second. The sample introduction flow velocity may be between 900 micrometres per second to 1000 micrometres per second.

The sample introduction flow velocity may be between 100 micrometres per second to 900 micrometres per second. The sample introduction flow velocity may be between 100 micrometres per second to 800 micrometres per second. The sample introduction flow velocity may be between 100 micrometres per second to 700 micrometres per second. The sample introduction flow velocity may be between 100 micrometres per second to 600 micrometres per second. The sample introduction flow velocity may be between 100 micrometres per second to 500 micrometres per second. The sample introduction flow velocity may be between 100 micrometres per second to 400 micrometres per second. The sample introduction flow velocity may be between 100 micrometres per second to 300 micrometres per second. The sample introduction flow velocity may be between 100 micrometres per second to 200 micrometres per second.

In one preferred example, the sample introduction flow velocity may be between 200 micrometres per second to 800 micrometres per second. In a particular, preferred, example, the sample introduction flow velocity may be 555 micrometres per second.

The sample introduction flow velocity may be greater than or equal to 1400 micrometres per second. In one highly preferred example, the sample introduction flow velocity may be greater than or equal to 2000 micrometres per second.

The sample introduction flow velocity may be provided for a duration of between 30 seconds and 5 minutes. Preferably, the sample introduction flow velocity may be provided for a duration of between 30 seconds and 2 minutes. Most preferably, the device sample introduction flow velocity may be provided for a duration of 1 minute. In another highly preferred embodiment, the sample introduction flow velocity may be provided for a duration of 30 seconds. However, the sample introduction flow velocity may be provided for a duration suitable to load the sample into the device.

The operational flow velocity may be greater than or equal to 20 micrometres per second. Preferably, the operational flow velocity is greater than or equal to 30 micrometres per second. Preferably still, the operational flow velocity is greater than or equal to 40 micrometres per second. Preferably still, the operational flow velocity is greater than or equal to 50 micrometres per second.

The operational flow velocity may be between 20 micrometres per second to 150 micrometres per second. The operational flow velocity may be between 30 micrometres per second to 150 micrometres per second. The operational flow velocity may be between 40 micrometres per second to 150 micrometres per second. The operational flow velocity may be between 50 micrometres per second to 150 micrometres per second. The operational flow velocity may be between 60 micrometres per second to 150 micrometres per second. The operational flow velocity may be between 80 micrometres per second to 150 micrometres per second. The operational flow velocity may be between 100 micrometres per second to 150 micrometres per second. The operational flow velocity may be between 120 micrometres per second to 150 micrometres per second. The operational flow velocity may be between 140 micrometres per second to 150 micrometres per second.

The operational flow velocity may be between 20 micrometres per second to 140 micrometres per second. The operational flow velocity may be between 20 micrometres per second to 120 micrometres per second. The operational flow velocity may be between 20 micrometres per second to 100 micrometres per second. The operational flow velocity may be between 20 micrometres per second to 80 micrometres per second. The operational flow velocity may be between 20 micrometres per second to 60 micrometres per second. The operational flow velocity may be between 20 micrometres per second to 50 micrometres per second. The operational flow velocity may be between 20 micrometres per second to 40 micrometres per second. The operational flow velocity may be between 20 micrometres per second to 30 micrometres per second.

In one preferred example, the operational flow velocity may be between 30 micrometres per second to 80 micrometres per second. In a particular, preferred, example, the operational flow velocity may be 55.5 micrometres per second. In another preferred example, the operational flow velocity is 55 micrometres per second.

In an example, controlling the fluid delivery arrangement to set the fluid flow to the operational flow velocity may comprises maintaining the fluid flow substantially at the operational flow velocity over a predetermined period of time.

The predetermined period of time may be greater than or equal to 1 minute. Preferably, the predetermined period of time is greater than or equal to 10 minutes. Preferably still, the predetermined period of time is greater than or equal to 15 minutes. Preferably still, the predetermined period of time is greater than or equal to 30 minutes.

The predetermined period of time may be greater than or equal to 1 minute. The predetermined period of time may be between 1 minute and 120 minutes. The predetermined period of time may be between 10 minutes to 45 minutes.

The predetermined period of time may be between 5 minutes and 60 minutes. The predetermined period of time may be between 10 minutes and 60 minutes. The predetermined period of time may be between 20 minutes and 60 minutes. The predetermined period of time may be between 30 minutes and 60 minutes. The predetermined period of time may be between 40 minutes and 60 minutes. The predetermined period of time may be between 50 minutes and 60 minutes.

The predetermined period of time may be between 5 minutes and 50 minutes. The predetermined period of time may be between 5 minutes and 40 minutes. The predetermined period of time may be between 5 minutes and 30 minutes. The predetermined period of time may be between 5 minutes and 20 minutes. The predetermined period of time may be between 5 minutes and 10 minutes.

In one preferred example, the predetermined period of time may be between 20 minutes to 40 minutes.

The method may further comprise controlling the fluid delivery arrangement to increase the fluid flow velocity from the operational flow velocity to a sample collection flow velocity higher than the operational flow velocity such that motile organisms are able to be collected from the sample collection zone. This controlling of the fluid delivery arrangement may be performed after the fluid flow velocity is maintained at the operational flow velocity for the predetermined period of time.

The sample collection flow velocity may be greater than or equal to 150 micrometres per second. In a particular, preferred, example, the sample collection flow velocity may be 250 micrometres per second. Preferably, the sample collection flow velocity is greater than or equal to 300 micrometres per second. Preferably still, the sample collection flow velocity is greater than or equal to 400 micrometres per second. Preferably, the sample collection flow velocity is greater than or equal to 500 micrometres per second. In a particular, preferred, example, the sample collection flow velocity may be 11000 micrometres per second.

The sample collection flow velocity may be between 150 micrometres per second and 15000 micrometres per second. The sample collection flow velocity may be between 400 micrometres per second and 11000 micrometres per second. The sample collection flow velocity may be between 400 micrometres per second and 10000 micrometres per second. The sample collection flow velocity may be between 500 micrometres per second and 10000 micrometres per second. In one highly preferred example, the sample collection flow velocity is 11100 micrometres per second.

The sample collection flow velocity may be between 150 micrometres per second and 8000 micrometres per second. The sample collection flow velocity may be between 150 micrometres per second and 6000 micrometres per second. The sample collection flow velocity may be between 150 micrometres per second and 4000 micrometres per second. The sample collection flow velocity may be between 150 micrometres per second and 2000 micrometres per second. The sample collection flow velocity may be between 150 micrometres per second and 1000 micrometres per second.

In one preferred example, the sample collection flow velocity may be between 400 micrometres per second and 700 micrometres per second. In one particular preferred example, the sample collection flow velocity may be 555 micrometres per second.

The sample collection flow velocity may be provided for a duration of between 0.5 second and 60 seconds. Preferably, the sample collection flow velocity may be provided for a duration of between 1 seconds and 20 seconds. Most preferably, the sample collection flow velocity may be provided for a duration of 4 seconds.

Prior to controlling the fluid delivery arrangement to set the fluid flow to have the sample introduction flow velocity, the method may comprise controlling the fluid flow to have a device priming flow velocity, wherein the device priming flow velocity is higher than the sample introduction flow velocity. The device priming flow velocity is selected to prime the device and ensure the channels, chambers and dead volumes are filled with fluid.

The device priming flow velocity may be greater than or equal to 800 micrometres per second. Preferably, the device priming flow velocity is greater than or equal to 1000 micrometres per second. Preferably, the device priming flow velocity is greater than or equal to 1200 micrometres per second. Preferably, the device priming flow velocity is greater than or equal to 1400 micrometres per second. Preferably, the device priming flow velocity is greater than or equal to or equal to 1500 micrometres per second. In a particular, preferred, example, the device priming flow velocity may be 1100 micrometres per second. A device may be primed via a device priming port. This may be achieved in a single step via the device priming port, through which the method disclosed herein may be implemented. The device priming flow velocity may be suitable to remove bubbles in the fluid.

The device priming flow velocity may be between 800 and 2000 micrometres per second. The device priming flow velocity may be between 1000 and 2000 micrometres per second. The device priming flow velocity may be between 1200 and 2000 micrometres per second. The device priming flow velocity may be between 1400 and 2000 micrometres per second. The device priming flow velocity may be between 1500 and 2000 micrometres per second.

The device priming flow velocity may be between 800 and 1800 micrometres per second. The device priming flow velocity may be between 800 and 1600 micrometres per second. The device priming flow velocity may be between 800 and 1400 micrometres per second. The device priming flow velocity may be between 800 and 1200 micrometres per second. The device priming flow velocity may be between 800 and 1000 micrometres per second.

In one preferred example, the device priming flow velocity may be between 1500 and 1600 micrometres per second. In one highly preferred example, the device priming flow velocity is 11100 micrometres per second.

The device priming flow velocity may be provided for a duration of between 1 second and 5 minutes. Preferably, the device priming flow velocity may be provided for a duration of between 5 seconds and 2 minutes. Most preferably, the device priming flow velocity may be provided for a duration of 1 minute. In one highly preferred example, the device priming flow velocity is provided for a duration of 6 seconds.

In one particular preferred implementation, the sample introduction flow velocity is between 200 micrometres per second to 800 micrometres per second, and the operational flow velocity is between 30 micrometres per second to 80 micrometres per second. In this preferred implementation, the flow velocity may be reduced from the sample introduction flow velocity to the operational flow velocity over a period of time of between 4 minutes and 20 minutes. In this preferred implementation, the operational flow velocity may be maintained for a period of between 20 minutes to 40 minutes. In this preferred implementation, the sample collection flow velocity may be between 400 micrometres per second and 700 micrometres per second. In this preferred implementation, the device priming flow velocity may be between 800 and 2000 micrometres per second, and is preferably between 1500 and 1600 micrometres per second.

The fluid delivery unit may comprise a fluid source and a pump. The pump may operate by suction or by pressure. The pump may be a microfluidic pump. The pump may be a syringe pump. The fluid source may be a syringe comprising fluid.

According to a second aspect of the present invention, there is provided a computer readable medium having instructions recorded thereon which, when executed by a computer, cause the computer to perform the method by controlling the fluid delivery unit as described above in relation to the first aspect of the invention.

According to a third aspect of the present invention, there is provided a sample separating apparatus arranged to provide a fluid flow to a device for separating motile organisms from other organisms. The device comprises a fluid inlet, a fluid terminus, a sample introduction zone and a sample collection zone. The apparatus comprises a fluid delivery unit; and a controller operable to control the fluid delivery unit to perform the method as described above in relation to the first aspect of the invention.

The sample separating apparatus may comprise one or more channels, each channel being defined by a pair of channel walls between the sample introduction zone and the sample collection zone. Each channel may extend radially outward from a central sample collection zone to the sample introduction zone. The sample introduction zone may therefore extend around the collection zone, for example in an arc or ring-like manner. The sample introduction flow velocity, and/or channel dimensions, may be set so that fluidic jets inhibit or prevent an organism in the sample from entering the channel. The operational flow velocity, and/or channel dimensions, may be set so that vortices are (e.g. turbulence is) induced proximal to an opening of the channel, the vortices aiding transportation of the organisms towards the channel opening.

Also disclosed herein is a sample separating device for separating motile organisms from other organisms. The device comprises a primary fluid path extending between a fluid inlet and a fluid terminus such that a fluid flow may be established between the fluid inlet and the fluid terminus. The device comprises a sample introduction zone disposed within the primary fluid path. The device comprises a secondary fluid path branching off from the primary fluid path at a location upstream of the sample introduction zone. The device comprises a sample collection zone disposed within or at a terminal end of the secondary fluid path. In use, motile organisms introduced into the sample introduction zone are able to swim against the fluid flow and enter the sample collection zone via the secondary fluid path.

Significantly, and in contrast to the device of Figure 1, the sample introduction zone is disposed within the primary fluid path between the fluid inlet and the fluid terminus. Further, the sample collection zone is outside of the primary fluid path, and is instead within or at the terminal end of a secondary fluid path that branches off from the primary fluid path at a location upstream of the sample introduction zone. This design of device exploits the motile organisms tendency to swim along the sides of the primary fluid path where the fluid travels at a lower velocity (e.g. because of a laminar flow being established in the primary fluid path). Because of this tendency, the motile organisms are able to follow the branched, secondary fluid path and enter the sample collection zone rather than swim towards the fluid inlet.

The primary fluid path may be defined by a first pair of channel walls between the sample introduction zone and the secondary fluid path. The primary fluid path may further be defined by a second pair of channel walls between the secondary fluid path and the fluid inlet. At least one of the first pair of the channel walls may extend from the sample introduction zone to the sample collection zone such that a continuous channel wall may be provided between the sample introduction zone and the sample collection zone. Significantly, a continuous channel wall surface may be provided between the sample introduction zone and the sample collection zone that motile organisms may swim or otherwise travel along. Particularly with motile organisms that exhibit thigmotaxis (such as sperm) this is beneficial as it allows the motile organisms to travel along the continuous wall surface from the sample introduction zone to the sample collection zone. This makes it easier for motile organisms to reach the sample collection zone without being lost in the fluid flow.

The secondary fluid path may branch off from the primary fluid path at a branching angle. The branching angle may be angle between a wall of the primary fluid path and a wall of the secondary fluid path.

The branching angle may be between 10 and 70 degrees. The branching angle may be between 20 and 70 degrees. The branching angle may be between 30 and 70 degrees. The branching angle may be between 40 and 70 degrees. The branching angle may be between 50 and 70 degrees. The branching angle may be between 60 and 70 degrees.

The branching angle may be between 10 and 60 degrees. The branching angle may be between 10 and 50 degrees. The branching angle may be between 10 and 40 degrees. The branching angle may be between 10 and 30 degrees. The branching angle may be between 10 and 20 degrees.

In one preferred example, the branching angle is 45 degrees.

The secondary fluid path may be curved. The secondary fluid path may be curved at an angle selected to ensure that a non-zero wall shear stress is experienced along the secondary fluid path. This means that rather than just branching off from the primary fluid path at a branching angle, the second fluid path is also curved with a curvature designed to sustain a wall shear stress along the wall in the curved region up to the sample collection zone. Without the curved region, the wall shear stress may decay to zero in the secondary fluid path. The non-zero wall stress means that motile organisms travelling along the wall of the secondary fluid path experience a non-zero wall shear stress. The non-zero wall stress may be beneficial in maximizing the amount of motile organisms that may progress along the continuous channel wall towards the sample collection zone, and may minimise the probability of motile organisms detaching from the wall and as a result not entering the sample collection zone. In other words, the motile organisms travelling near the curved wall will continue to progress because they experience fluidic forces. The use of a curved secondary fluid path that provides a non-zero wall shear stress may be beneficial in reducing the time required for collecting sufficient motile organisms in the sample collection zone.

The device may comprise two secondary fluid paths. Both of the secondary fluid paths may branch off from the primary fluid path at locations upstream of the sample introduction zone. The two secondary fluid paths may branch off from the primary fluid path at the same location. The two secondary fluid paths may branch off from opposed walls of the primary fluid path. The two secondary fluid paths may be mirror images of one another. The device may comprise two sample collection zones each disposed within or at a terminal end of a respective one of the secondary fluid paths. Alternatively, a single sample collection zone may be provided that may be shared by both of the secondary fluid paths. The single sample collection zone may be in the form of a channel that connects the two secondary fluid paths together. The single sample collection zone beneficially simplifies the process of collecting the samples.

One of the first pair of the channel walls may extend from the sample introduction zone to the sample collection zone of one of the secondary fluid paths such that a continuous channel wall is provided between the sample introduction zone and the sample collection zone. The other of the first pair of the channel walls may extend from the sample introduction zone to the sample collection zone of the other one of the secondary fluid paths such that a continuous channel wall is provided between the sample introduction zone and the sample collection zone.

The device may comprise at least one air permeable, fluid impermeable membrane (a semi-permeable membrane). The membrane may cover the sample collection zone. The membrane may allow air to escape from the device as fluid is provided to the device, and thus prevents trapped air from remaining in the fluid paths of the device. Trapped air may block the fluid paths of the device and prevent motile organisms from progressing, and thereby may compromise the efficacy of the device. The semi-permeable membrane allows the air to escape but prevents fluid for escaping.

The device may comprise a valve. The valve may operate to obstruct the primary fluid path so as to isolate the sample collection zone from the sample introduction zone. This effectively isolates the motile organisms from the sample collection zone from the initial sample remaining in the sample introduction zone. The valve may be a cut-off valve.

A region of the primary fluid path proximate to the sample introduction zone may taper outwards. This may mean that the cross-sectional area of the primary fluid path proximate to the sample introduction zone is greater than other portions of the primary fluid path. The primary fluid path may taper outwards in two spatial dimensions or in three spatial dimensions. The tapering of the primary fluid path may be beneficial in reducing the local flow velocity in the vicinity of the sample introduction zone, and in increasing the cross-sectional area of the inlet to the sample introduction zone. Beneficially, this may expose more of the sample to a the flow, thereby increasing the number of organisms that move against the flow (e.g. via taxis). This may improve the yield in the sample collection zone.

A region of the secondary fluid path proximate to the sample collection zone may taper inwards. This may mean that the cross-sectional area of the secondary fluid path proximate to the sample collection zone is smaller than other portions of the secondary fluid path. The secondary fluid path may taper inwards in two spatial dimensions or in three spatial dimensions. The narrowing of the secondary fluid path may be beneficial in preventing or making it harder for motile organisms within the sample collection zone to move back out of the sample collection zone and into the secondary fluid path.

The device may be a microfluidic device. The primary fluid path between the secondary fluid path and the sample introduction zone may be a microchannel. The primary fluid path between the fluid inlet and the sample introduction zone may be a microchannel. The primary fluid path between the fluid inlet and the fluid terminus may be a microchannel. The secondary fluid path may be or comprise a microchannel. The device may comprise a substrate. The microchannel may be provided on the substrate.

The microchannel may have a width of less than or equal to 1000 micrometres. Preferably, the width of the microchannel is less than or equal to 800 micrometres. Preferably still, the width of the microchannel is less than or equal to 600 micrometres. Preferably still, the width of the microchannel is less than or equal to 400 micrometres. Preferably still, the width of the microchannel is less than or equal to 200 micrometres. Preferably still, the width of the microchannel is less than or equal to 100 micrometres.

The width of the microchannel may be between 10 micrometres to 1000 micrometres. The microchannel may have a width of between 20 micrometres to 1000 micrometres. The microchannel may have a width of between 50 micrometres to 1000 micrometres. The microchannel may have a width of between 100 micrometres to 1000 micrometres. The microchannel may have a width of between 200 micrometres to 1000 micrometres. The microchannel may have a width of between 400 micrometres to 1000 micrometres. The microchannel may have a width of between 600 micrometres to 1000 micrometres. The microchannel may have a width of between 800 micrometres to 1000 micrometres.

The width of the microchannel may be between 10 micrometres to 800 micrometres. The microchannel may have a width of between 10 micrometres to 600 micrometres. The microchannel may have a width of between 10 micrometres to 400 micrometres. The microchannel may have a width of between 10 micrometres to 200 micrometres. The microchannel may have a width of between 10 micrometres to 100 micrometres. The microchannel may have a width of between 10 micrometres to 50 micrometres. The microchannel may have a width of between 10 micrometres to 20 micrometres.

The microchannel may have a height of greater than or equal to 10 micrometres. Preferably, greater than or equal to 20 micrometres. Preferable still, greater than or equal to 30 micrometres. Preferably still, greater than or equal to 40 micrometres. Preferably still, greater than or equal to or equal to 50 micrometres.

The height of the microchannel may be between 10 micrometres to 450 micrometres. The height of the microchannel may be between 20 micrometres to 450 micrometres. The height of the microchannel may be between 30 micrometres to 450 micrometres. The height of the microchannel may be between 40 micrometres to 450 micrometres. The height of the microchannel may be between 50 micrometres to 450 micrometres. The height of the microchannel may be between 100 micrometres to 450 micrometres. The height of the microchannel may be between 200 micrometres to 450 micrometres. The height of the microchannel may be between 300 micrometres to 450 micrometres. The height of the microchannel may be between 400 micrometres to 450 micrometres.

The height of the microchannel may be between 10 micrometres to 400 micrometres. The height of the microchannel may be between 10 micrometres to 300 micrometres. The height of the microchannel may be between 10 micrometres to 200 micrometres. The height of the microchannel may be between 10 micrometres to 100 micrometres. The height of the microchannel may be between 10 micrometres to 50 micrometres. The height of the microchannel may be between 10 micrometres to 40 micrometres. The height of the microchannel may be between 10 micrometres to 30 micrometres. The height of the microchannel may be between 10 micrometres to 20 micrometres.

In one preferred example, the height of the microchannel is between 50 micrometres to 150 micrometres.

The primary fluid path may have a length greater than or equal to 1 millimetre. Preferably, the length of the primary fluid path is greater than or equal to 2 millimetres. Preferably, the length of the primary fluid path is greater than or equal to 3 millimetres. Preferably, the length of the primary fluid path is greater than or equal to 4 millimetres. Preferably, the length of the primary fluid path is greater than or equal to 5 millimetres.

The length of the primary fluid path may be between 1 millimetre to 40 millimetres. The length of the primary fluid path may be between 5 millimetres to 40 millimetres. The length of the primary fluid path may be between 10 millimetres to 40 millimetres. The length of the primary fluid path may be between 20 millimetres to 40 millimetres. The length of the primary fluid path may be between 30 millimetres to 40 millimetres.

The length of the primary fluid path may be between 1 millimetre to 30 millimetres. The length of the primary fluid path may be between 1 millimetre to 20 millimetres. The length of the primary fluid path may be between 1 millimetre to 10 millimetres. The length of the primary fluid path may be between 1 millimetre to 5 millimetres.

In one preferred example, the primary fluid path has a length of between 5 millimetres to 10 millimetres.

The device may comprises an attachment interface arranged to attach the device to a fluid delivery unit for delivering a fluid flow to the fluid inlet.

The device may comprise a plurality of primary fluid paths extending between the fluid inlet and the fluid terminus. The use of a plurality of primary fluid paths may enable a greater yield of motile organisms to be obtained. The example of the device with a single primary fluid path may enable around 200-1000 sperm cells to reach the sample collection zone for selection by an embryologist. This is ideally suited for ICSI processes. The example of the device with a plurality of primary fluid paths may enable a greater yield of over 10000 sperm cells to reach the sperm collection zone or zones. This may be more suited to conventional IVF applications.

The device may comprise a plurality of secondary fluid paths each branching off a respective one of the primary fluid paths at a location upstream of the sample introduction zone. The sample introduction zone may be disposed within the plurality of primary fluid paths. The sample introduction zone may comprise one sample introduction zone or a plurality of sample introduction zones which may each be associated with one of the primary fluid paths. Two secondary fluid paths may branch off from each of the primary fluid paths. The sample collection zone may be disposed within or at a terminal end of the plurality of secondary fluid paths.

A plurality of sample collection zones may be provided where each of the sample collection zones is associated with one or a subset of the secondary fluid paths. For example, two secondary fluid paths may branch off form each of the primary fluid paths and each of the two secondary fluid paths may be associated with a different sample collection zone.

A single sample collection zone may be provided. The single sample collection zone may be shared by all of the secondary fluid paths.

The number of primary fluid paths may be greater than or equal to 5. Preferably, greater than or equal to 10.

The number of primary fluid paths may be between 5 and 100. The number of primary fluid paths may be between 10 and 100. The number of primary fluid paths may be between 20 and 100. The number of primary fluid paths may be between 30 and 100. The number of primary fluid paths may be between 40 and 100. The number of primary fluid paths may be between 50 and 100. The number of primary fluid paths may be between 60 and 100. The number of primary fluid paths may be between 70 and 100. The number of primary fluid paths may be between 80 and 100. The number of primary fluid paths may be between 90 and 100.

The number of primary fluid paths may be between 5 and 90. The number of primary fluid paths may be between 5 and 80. The number of primary fluid paths may be between 5 and 70. The number of primary fluid paths may be between 5 and 60. The number of primary fluid paths may be between 5 and 50. The number of primary fluid paths may be between 5 and 40. The number of primary fluid paths may be between 5 and 30. The number of primary fluid paths may be between 5 and 20. The number of primary fluid paths may be between 5 and 10.

In a preferred example, the number of primary fluid paths is between 6 and 24, and is preferably 12.

The device may further comprise an identifier. The identifier may be a unique identifier. The identifier may be in the form a printed identifier code such as a barcode or QR code. The identifier may be in the form of an RFID tag.

The device may further comprise a cell counter. The cell counter may be used to indicate the concentration of motile organisms in the sample collection zone and/or provide an assessment of the motility of the organisms. The cell counter may have a fixed volume. Organisms in the sample collection zone may be drawn into the cell counter by capillary action. The cell counter may have a grid arrangement to aid in the manual counting of organisms within the cell counter.

The device may be used in the method as described above in relation to the first aspect of the invention. In other words, a fluid delivery unit may be controlled to provide a fluid flow to the sample separating device disclosed herein so as to provide a fluid flow between the fluid inlet and the fluid terminus of the device. The fluid flow may have a sample introduction flow velocity set so that a sample may be introduced into the sample introduction zone of the device. The sample introduction flow velocity may be sufficiently high such that an organism in the sample is unable to exit the sample introduction zone. The fluid delivery unit may be controlled to reduce the fluid flow velocity from the sample introduction flow velocity to an operational flow velocity lower than the sample introduction flow velocity. The operational flow velocity may be selected such that motile organisms in the sample are able to swim against the fluid flow and enter a sample collection zone of the device.

Also disclosed herein is a sample separating method for separating motile organisms from other organisms. The method comprises providing a device as described above. The method comprises introducing a fluid to the fluid inlet of the device such that a fluid flow is established between the fluid inlet and the fluid terminus.

The method may comprise the method as described above in relation to the first aspect of the present invention.

Also disclosed herein is a system comprising: a sample separating device as described above; and a fluid delivery unit arranged to deliver a fluid flow to the fluid inlet of the device.

Also disclosed herein is a sample separating apparatus for separating motile organisms from other organisms. The apparatus comprises a device reception unit arranged to receive a device. The device comprises a fluid inlet, a fluid terminus, a sample introduction zone, and a sample collection zone. The apparatus comprises a fluid delivery unit in fluid communication with the device reception unit and arranged to deliver a fluid to the fluid inlet of the device such that a fluid flow may be established between the fluid inlet and the fluid terminus of the device. The apparatus comprises a sample introduction unit arranged to introduce a sample to the sample introduction zone of the device such that motile organisms introduced into the sample introduction zone are able to swim against the fluid flow and enter the sample collection zone.

Significantly, the sample separating apparatus allows a device to be loaded into the apparatus. Fluid is then delivered to the device, and a sample is then delivered to the sample introduction zone of the device. The delivery of fluid and the introduction of the sample may be an automated process that is user independent.

The fluid delivery unit may be an automated fluid delivery unit arranged to deliver fluid without requiring a specific user input. The sample introduction unit may be an automated sample introduction unit arranged to deliver a sample without requiring a specific user input. The sample introduction unit may be arranged to introduce a pre-defined volume of sample to the sample introduction zone of the device.

The apparatus may be arranged to enclose the device. This may protect the device from environmental disturbances during the sample separating operation. Enclosing the device within the apparatus may have the effect of sealing the device from local atmospheric pressures. Local atmospheric pressure changes can be caused by acts as simple as opening or closing a door in the laboratory where the apparatus is located. That is, when the device is enclosed in the apparatus, the apparatus is arranged to seal the device from atmospheric pressure changes in the vicinity of the apparatus.

The device reception unit may be arranged to transition between an open configuration in which a device may be received and a closed configuration in which the device is enclosed within the apparatus.

The apparatus may be arranged to control against environmental disturbances. That is, the apparatus may provide a controlled environment for the sample separating operation. The environmental disturbances may comprise at least one of vibration, atmospheric pressure changes, and temperature changes. The apparatus may thus be an automated system that helps ensure consistent, controlled and reliable implementation of a sample separating process using a sample separating device loaded into the apparatus. The apparatus enables temperature fluctuations, pressure fluctuations, or other environmental source of noise which may comprise the efficacy of the sample separating device to be controlled.

The apparatus may comprise a temperature control unit operable to control the temperature within at least the vicinity of the device reception unit of the apparatus. The temperature control unit may comprise a temperature sensor. The temperature sensor may be arranged to sense the temperature within the vicinity of the device reception unit. The temperature control unit may be arranged to control (e.g. increase or decrease) the temperature in the vicinity of the device reception unit in response to the temperature sensor sensing a temperature outside of a range of preferred temperature values. The temperature control unit may comprise a heating and/or cooling unit. In a particular example, the temperature control unit may be operable to maintain the temperature at 37 degrees Centigrade.

The apparatus may comprise a vibration suppression arrangement for supressing vibration of at least the device when received in the device reception unit. The vibration suppression arrangement may comprise vibration damping material. The vibration suppression arrangement may comprise a vibration isolation system arranged to isolate the device, when received in the device reception unit, from external vibrations. The vibration isolation system may comprise a passive isolation system. The passive isolation system may comprise a spring, a pneumatic air isolator, or a cushioning, flexible, material such as an elastomeric material. The vibration isolation system may comprise an active isolation system. The active isolation system may comprise a vibration sensor, a controller, and an actuator. The vibration sensor may sense vibration which may be processed by the controller and used to actuate an actuator for supressing vibration. The actuator may be a linear actuator, a pneumatic actuator or a piezoelectric actuator. The sensor may be an accelerometer.

The sample introduction unit may be arranged to introduce pre-defined volume of sample to the sample introduction zone. The sample introduction unit may be arranged to introduce a sample to the sample introduction zone of the device automatically in response to at least one predetermined condition being reached.

The at least one predetermined condition may comprise at least one of a predetermined time having elapsed after the device is received by the device reception unit, a predetermined flow velocity being established, and a predetermined environmental condition within at least the vicinity of the device reception unit of the apparatus being established. The environmental condition may be a temperature condition.

The apparatus may further comprise a sample collection unit arranged to recover the sample from the sample collection zone. The sample collection unit may be an automated sample collection unit arranged to collect a sample without requiring a specific user input. The sample collection unit may be arranged to transfer the collected sample to a receptacle.

The sample collection unit may be arranged to recover the sample from the sample collection zone of the device automatically in response to at least one predetermined condition being reached.

The at least one predetermined condition may comprise at least one of a predetermined time having elapsed after the sample is introduced into the sample introduction zone by the sample introduction unit, and a predetermined flow velocity being established.

The apparatus may further comprise a controller operable to control the fluid delivery unit. The controller may be operable to control the sample introduction unit. The controller may be operable to control the sample collection unit. The controller may be operable to control the vibration suppression arrangement and/or the temperature control unit.

The apparatus may further comprise an imaging unit operable to image the device received in the device reception unit. The imaging unit may thus allow for optical monitoring of the sample. Preferably, the imaging unit allows for optical monitoring of the sample collection zone such that a user is able to ascertain whether sufficient motile organisms have entered the sample collection zone. The imaging unit may be operable to magnify the sample for visual inspection. The imaging unit may be operable to use phase contrast to more clearly visually highlight the sample within the device. The imaging unit may be moveable so as to change to location of the device which is imaged. The imaging unit may be motorised, and may be moveable within a 2D plane above the device. The imaging unit may be moveable by approximately 15 millimetres in the X direction of the 2D plane, and approximately 10 mm in the Y direction of the 2D plane.

The device may be a microfluidic device. The device may be a device as described above. The controller may be operable to control the fluid delivery unit to deliver fluid to the device in accordance with the first aspect of the present invention.

Also disclosed herein is a method of separating motile organisms from other organisms using a sample separating apparatus. The method comprises positioning a device in a device reception unit of the sample separating apparatus. The device comprises a fluid inlet, a fluid terminus, a sample introduction zone, and a sample collection zone. The method comprises controlling a fluid delivery unit of the sample separating apparatus to deliver a fluid to the fluid inlet of the device such that a fluid flow may be established between the fluid inlet and the fluid terminus of the device. The method comprises controlling a sample introduction unit of the sample separating apparatus to deliver a sample to the sample introduction zone of the device such that motile organisms introduced into the sample introduction zone are able to swim against the fluid flow and enter the sample collection zone.

The method may comprise controlling the fluid delivery unit to provide a fluid flow having a sample introduction flow velocity. The sample introduction velocity being set so that a sample may be introduced into a sample introduction zone of the device. While the fluid flow has the sample introduction flow velocity, the method may comprise controlling the sample introduction unit to deliver the sample to the sample introduction zone.

The method may comprise reducing the flow velocity to an operational flow velocity after the sample is delivered to the sample introduction zone. The operational flow velocity being selected such that motile organisms in the sample are able to swim against the fluid flow and enter a sample collection zone of the device.

The method may comprise controlling a sample collection unit of the sample separating apparatus to collect a sample from a sample collection zone of the device. The method may comprise increasing the flow velocity to a sample collection flow velocity before controlling the sample collection unit to collect the sample from the sample collection zone.

The method may comprise controlling the fluid delivery unit according to the approaches described above in relation to the first aspect of the invention.

In all aspects of the present invention, the fluid refers to a medium suitable for the desired motile organisms to move through. The medium may be a cell medium. The fluid may be a liquid and may a buffer solution. The fluid may be a saline solution or a more viscous solution.

In all aspects of the present invention, the motile organisms may be organisms that exhibit a form of taxis. Taxis will be understood as the ability of certain organisms to move in response to a stimulus. The motile organisms may exhibit one or a combination of rheotaxis, thigmotaxis, chemotaxis, and thermotaxis. Rheotaxis will be understood as meaning the ability of certain organisms to generally align with and swim against a fluid flow. Thigmotaxis will be understood as meaning the ability of certain organisms travel to move or in the vicinity of a channel wall. Chemotaxis will be understood as meaning the ability of certain organisms to move in response to a chemical stimulus. Thermotaxis will be understood as meaning the ability of certain organisms to move along or against a temperature gradient.

In all aspects of the present invention, the motile organisms may be sperm. The sample may be a sperm sample. The sperm sample may comprise motile sperm, dead sperm, and deformed or otherwise non-motile sperm. The sperm sample may also comprise other organisms such as white blood cells which are non motile and are ideally separated from the sperm sample before an artificial insemination operation is performed. The present invention is not limited to sperm separation. Other organisms that are able to move against a fluid flow may be separated according to approaches of the present invention. The organisms could be bacterial or eukaryotic cells with flagellums that enable them to swim against a fluid flow.

For a better understanding of the invention, and to show how embodiments of the same may be carried into effect, reference will now be made, by way of example only, to the accompanying diagrammatic drawings in which:
Figure 1 is a schematic diagram of a sample separating device according to an existing implementation;
Figure 2 is a process flow diagram of an example sample separating method according to aspects of the present invention;
Figure 3 is another process flow diagram of an example sample separating method according to aspects of the present invention;
Figure 4(a) is a plot showing the change in fluid flow velocity against time according to an example sample separating method according to aspects of the present invention;
Figure 4(b) is a graph showing the number of unsorted sperm in the sample collection zone against period of time for reduction from the sample introduction flow velocity to the operational flow velocity;
Figures 5(a)-5(c) are schematic diagrams of an example sample separating device disclosed herein;
Figure 6 is a schematic diagram of another example sample separating device disclosed herein;
Figure 7 is a schematic diagram of another example sample separating device disclosed herein;
Figures 8(a) to 8(b) are schematic diagrams of yet another example sample separating device disclosed herein;
Figure 8(c) is a schematic diagram of another example sample separating device disclosed herein;
Figure 9 is a schematic diagram of another example sample separating device disclosed herein;
Figure 10 is a simplified block diagram of an example sample separating apparatus disclosed herein; and
Figures 11(a)-11(b) are perspective views of another example sample separating apparatus disclosed herein.

Referring to Figure 2, there is shown an example process flow diagram for a method according to an example implementation of the present invention. The method is a sample separating method for separating motile organisms from other organisms.

Step 201 of the method comprises providing a fluid flow having a sample introduction flow velocity. In particular, the method comprises controlling a fluid delivery unit to provide a fluid flow to a sample separating device comprising a fluid inlet and a fluid terminus. The fluid flow is provided between the fluid inlet and the fluid terminus of the device and may be referred to as the primary fluid path. In other words, the device provides a channel between the fluid inlet and the fluid terminus of the device. The fluid delivery unit is controlled to delivery fluid to the fluid inlet such that it may flow through the channel to the fluid terminus.

The sample introduction flow velocity is set so that a sample may be introduced into a sample introduction zone of the device. The sample may be introduced into the sample introduction zone by, for example, pipetting the sample into the sample introduction zone. The sample may be introduced by a sample introduction unit of a sample separating apparatus that the device is positioned within.

Importantly, the sample introduction flow velocity is set to be sufficiently high such that an organism in the sample is unable to exit the sample introduction zone. By this, it is meant that the flow velocity of the fluid flow is sufficiently high that the fluid flow between the fluid inlet and fluid terminus presents a barrier that prevents organisms within the sample introduction zone from leaving the sample introduction zone and entering the fluid flow. This, for example, prevents organisms for being wicked up the conduit towards a sample collection zone of the device by capillary action.

Step 202 of the method comprises reducing the fluid flow velocity to an operational flow velocity lower than the sample introduction velocity. In particular, the method comprises controlling the fluid delivery unit to reduce the fluid flow velocity from the sample introduction flow velocity to an operational flow velocity lower than the sample introduction flow velocity. The operational flow velocity is selected such that motile organisms in the sample are able to swim against the fluid flow and enter a sample collection zone of the device.

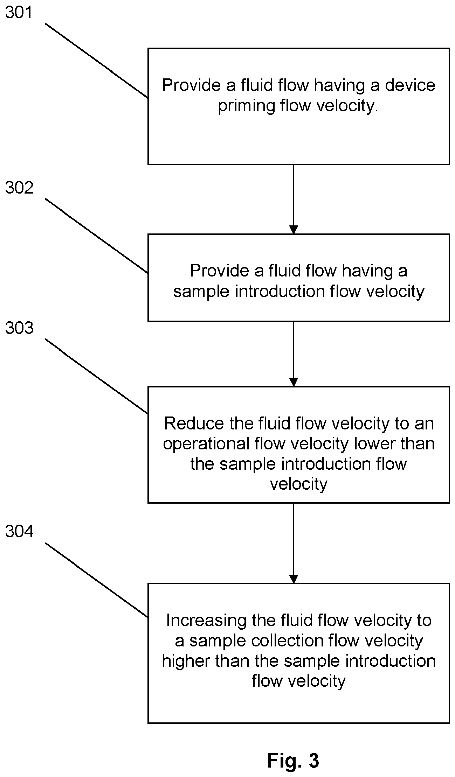

Referring to Figure 3, there is shown another example process flow diagram for a method according to an example implementation of the present invention. The method is a sample separating method for separating motile organisms from other organisms.

Step 301 of the method comprises providing a fluid flow having a device priming flow velocity.

Step 302 of the method comprises providing a fluid flow having a sample introduction flow velocity. In other words, the flow velocity is reduced from the device priming flow velocity to the sample introduction flow velocity. The sample introduction flow velocity is lower than the device priming flow velocity.

Step 303 of the method comprises reducing the fluid flow velocity to an operational flow velocity lower than the sample introduction flow velocity. The fluid flow velocity is maintained at the operational flow velocity for a period of time sufficient to allow a desired quantity of motile organisms to swim against the fluid flow and enter the sample collection zone.

Step 304 of the method comprises increasing the fluid flow velocity to a sample collection flow velocity higher than the sample introduction flow velocity. This means that once a sufficient concentration of motile organisms are in the sample collection zone, the flow velocity may be increased to a sample collection flow velocity. When the flow velocity is at the sample collection flow velocity, the sample may be removed from the sample collection zone. In particular, a sample collection unit of the sample separating apparatus may be controlled to remove the sample from the sample collection zone. The sample collection flow velocity may be set to be sufficiently high to prevent organisms within the sample introduction zone from being drawn to the sample collection zone as the sample is being removed from the sample collection zone.

In an exemplary embodiment of a method in accordance with the present invention, the method further comprises a bubble removal step. In an exemplary embodiment, the bubble removal step forms part of, or is comprised in, the device priming step. That is, device priming step 301 comprises bubble removal. The bubble removal step precedes the introduction of the sample. In this way, bubbles may be removed from the fluid before sample introduction. Bubble removal may be facilitated by providing a fluid flow at a bubble removal flow velocity. Bubble location is a function of device architecture, degassing, air ingress, material and fluid and air interactions (including surface tensions).

Referring to Figure 4(a), there is shown an example plot of the flow velocity against time according to an example implementation of the method described above in relation to Figure 3. The y-axis of the plot represents the velocity and uses a logarithmic scale. The x-axis of the plot represents the time and uses a linear scale.

Figure 4(a) shows that the process starts by providing fluid flow at the device priming flow velocity 401. The device priming flow velocity is 1.6 millimetres per second in this example. The fluid delivery unit may generate fluid flow at the device priming flow velocity for a short period of time. The short period of time may even be between 1 second and 2 seconds.

The device priming flow velocity 401 is then reduced to the sample introduction flow velocity 402. The reduction of the flow velocity from the device priming flow velocity 401 to the sample introduction flow velocity 402 may happen over a short period of time, and may appear to occur instantaneously as shown in Figure 4(a). The fluid flow velocity may be set at the sample introduction flow velocity 402 for around 1 minute

The sample introduction flow velocity 402 is then reduced to the operational flow velocity 403. The reduction of the flow velocity from the sample introduction flow velocity 402 to the operational flow velocity 403 happens gradually, and in the example of Figure 4(a) takes approximately 10 minutes. The fluid delivery unit then maintains the operational flow velocity 403 for a relatively long period of time. This period of time may be the time taken for sufficient motile organisms to arrive at the sample collection zone of the device. This may be confirmed by visual inspection of the sample collection zone. Generally, the fluid delivery unit will maintain the operational flow velocity 403 for between 20 minutes and 40 minutes. In the particular example shown in Figure 4(a), the operational flow velocity 403 is maintained for 20 minutes.

The operational flow velocity 403 is then increased to the sample collection flow velocity 404. The sample collection flow velocity 404 in this example is 555 micrometres per second. The flow velocity is maintained at the sample collection flow velocity 404 for 5 minutes. Once the sample collection flow velocity 404 is established, the sample and other fluid in the sample introduction zone may be collected and discarded. Further, the sample within the sample collection zone may be collected.

Referring to Figure 4(b), the effect of reducing the sample introduction flow velocity 402 to the operational flow velocity 403 over a predetermined period of time is quantified with reference to the following non-limiting examples provided below. Whilst the examples provided relate to a reduction from a sample introduction flow velocity 402 of 1400 micrometres per second to an operational flow velocity 403 of 55 micrometres per second over 0, 10, 20 and 30 seconds, the person skilled in the art will appreciate that similar advantages are obtainable using different time periods or flow velocities.

The y-axis indicates the number of unsorted sperm which reached the sample collection zone. The x-axis indicates the period of time taken for the reduction of the flow velocity from the sample introduction flow velocity 402 to the operational flow velocity 403.

As shown in the "0 second" column, reducing the sample introduction flow velocity 402 to the operational flow velocity 403 instantaneously (i.e. abruptly, suddenly, or without a gradual reduction over a period of time) results in a large number of unsorted sperm reaching the sample collection zone. This is highly undesirable.

As shown in the "10 second" column, reducing the sample introduction flow velocity 402 to the operational flow velocity 403 over a period of 10 seconds (i.e. a gradual reduction, over a pre-determined period of time, in a non-abrupt manner) dramatically reduces the number of unsorted sperm which are able to reach the sample collection zone. This is highly desirable and advantageous.

As shown in the "20 second" column, reducing the sample introduction flow velocity 402 to the operational flow velocity 403 over a period of at least 20 seconds (i.e. a gradual reduction, over a pre-determined period of time, in a non-abrupt manner) results in a further dramatic reduction of unsorted sperm which are able to reach the sample collection zone over both the "0 second" and the "10 second" time periods.

As shown in the "30 second" column, reducing the sample introduction flow velocity 402 to the operational flow velocity 403 over a period of 30 seconds (i.e. a gradual reduction, over a pre-determined period of time, in a non-abrupt manner) results in no unsorted sperm being able to reach the sample collection zone. This is highly desirable and provides for optimal sperm sorting (i.e. where dead sperm, and deformed or otherwise non-motile sperm are unable to reach or be found in the collected sample in the sample collection zone).

So, from the above it can be understood that when the flow velocity is reduced in an abrupt or instantaneous manner, unsorted sperm inadvertently reach the sample collection zone. Reducing the flow velocity over a predetermined period of time reduces the number of unsorted sperm present in the collected sample. Indeed, increasing the period of time over which the flow velocity is reduced can prevent some or all (in this case, by reducing over 30 seconds) unsorted sperm reaching the sample collection zone. This is highly advantageous in optimising the quality of the collected sample.

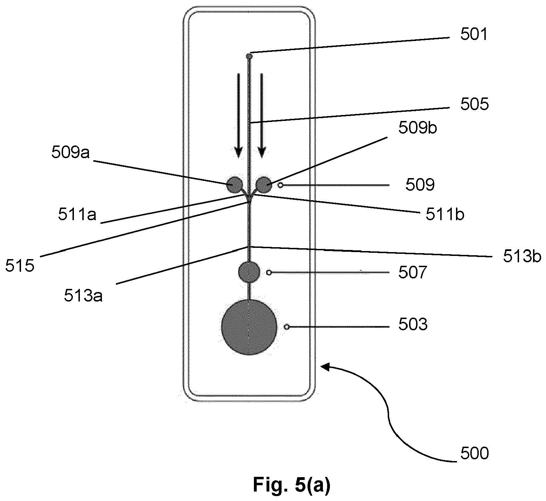

Referring to Figures 5(a) to 5(c), there is shown a sample separating device 500 for separating motile organisms from other organisms. The sample separating device 500 is in the form of a microfluidic chip 500.

The device 500 comprises a fluid inlet 501. The fluid inlet 501 denotes a point where fluid may enter the device from a fluid delivery unit such as a pump in cooperation with a fluid store. The device 500 also comprises a fluid terminus 503 in fluid communication with the fluid inlet 501. The fluid terminus 503 in this example is in the form of a fluid collection well 503. A microfluidic channel 505 extends between the fluid inlet 501 and the fluid terminus 503. Fluid flowing from the fluid inlet 501 to the fluid terminus 503 forms a primary fluid path 505 for the device 500.

The device 500 comprises a sample introduction zone 507. The sample introduction zone 507 denotes a region of the device 500 where a sample may be introduced. This is typically performed by pipetting the sample into the sample introduction zone 507. The sample introduction zone 507 is provided within the primary fluid path 505 at a location between the fluid inlet 501 and the fluid terminus 503, and in particular is proximate to the fluid terminus 503.

The device 500 comprises a sample collection zone 509. The sample collection zone 509 denotes a region of the device 500 where motile organisms may be collected. The sample collection zone 509 is located upstream of the sample introduction zone 507. In the example of Figure 5(a), two sample collection zones 509a, 509b are provided at the terminal ends of microchannels 511a, 511b that have branched off of the primary flow path 505. The microchannels 511a, 511b form secondary fluid paths 511a, 511b.

In more detail, it can be seen that the primary fluid path 505 is defined by opposed side walls 513a, 513b. At the branching point 515, the two side walls 513a, 513b branch off to form side walls of the microchannels 511a, 511b. In this way, there is a continuous wall surface extending between the sample introduction zone 507 and each of the sample collection zones 509a, 509b.

Figure 5(a) shows the device 500 during step 301 of the method described above in relation to Figure 3. In particular, a fluid flow is provided between the fluid inlet and the fluid terminus having the device priming flow velocity. At this stage, no sample has been introduced into the sample introduction zone 507.

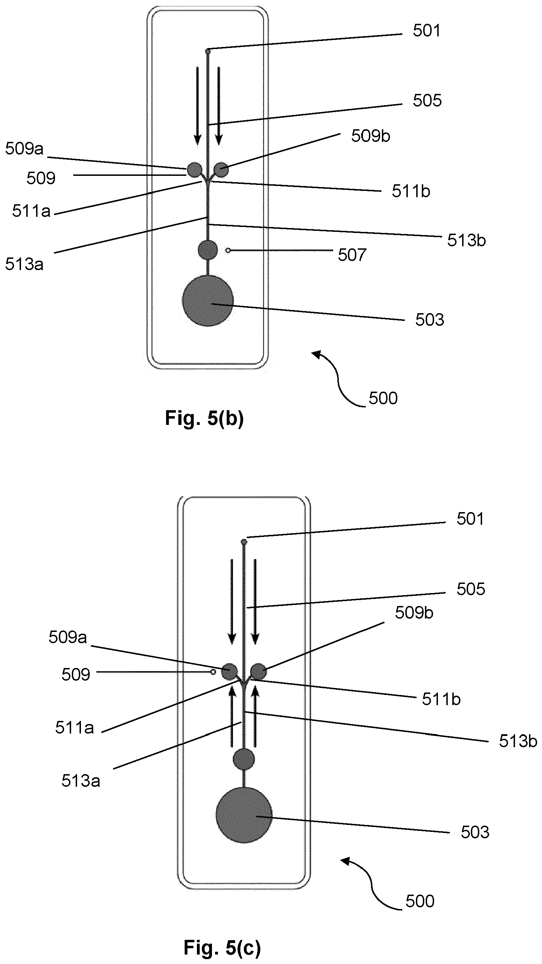

Figure 5(b) shows the device 500 during step 302 of the method described above in relation to Figure 3. In particular, the fluid flow velocity has been reduced to the sample introduction flow velocity. In addition, a sample has been pipetted into the sample introduction zone 507.

Figure 5(c) shows the device 500 during step 303 of the method described above in relation to Figure 3. In particular, the fluid flow velocity has been reduced to the operational flow velocity. Motile organisms are able to swim against the fluid flow, escape the sample introduction zone 507 and enter the sample collection zone 509.

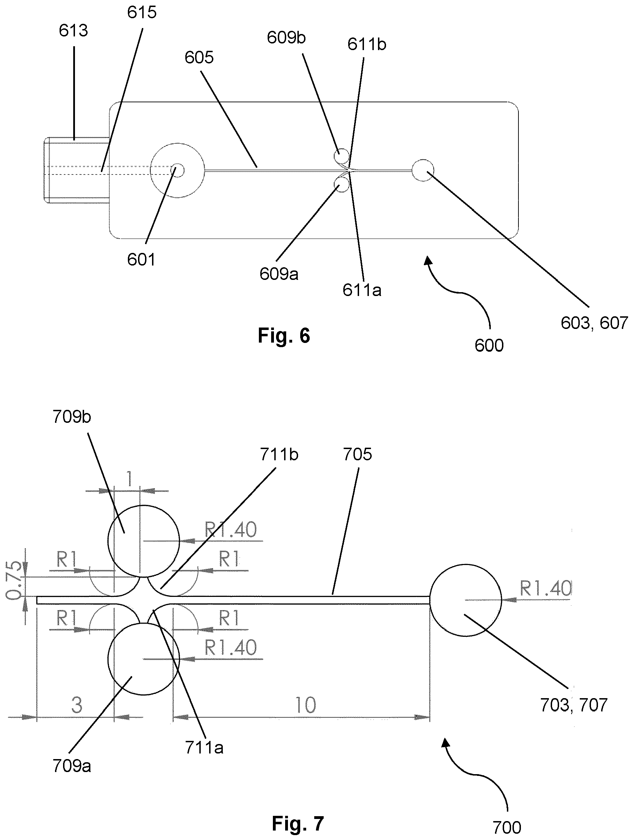

Referring to Figure 6, there is shown another example sample separating device 600.

The sample separating device 600 comprises a fluid inlet 601. A connector in the form of a push connector 613 is provided upstream of the fluid inlet 601. The push connector 613 comprises a channel 615 which is in fluid communication with the fluid inlet 601. The push connector 613 enables the sample separating device 600 to be connected to a fluid delivery unit.

The sample separating device 600 further comprises a fluid terminus 603 in the form of a fluid outlet 603. Fluid is able to flow out of the sample separating device 600 via the fluid outlet 603. A sample introduction zone 607 is provided in the vicinity of the fluid outlet 603. A primary fluid path 605 extends between the fluid inlet 601, sample introduction zone 607 and fluid outlet 603.

The sample separating device 600 further comprises two sample collection zone 609a, 609b located upstream of the sample introduction zone 607. The two sample collection zones 609a, 609b are provided at the terminal ends of microchannels 611a, 611b that have branched off of the primary flow path 605. The microchannels 611a, 611b form secondary fluid paths 611a, 611b.

Referring to Figure 7, there is shown another example of primary and second flow paths 705, 711a, 711b in a sample separating device 700.

The primary flow path extends between a fluid inlet (not shown) and a sample introduction zone/fluid terminus 707, 703. Two secondary fluid paths 711a, 711b branch off from the primary flow path 705 and terminate in sample collection zones 709a, 709b. The secondary fluid paths 711a, 711b in this example are curved at an angle to ensure that a non-zero wall shear stress is experienced along the secondary fluid path. In this particular example, the curved secondary fluid paths 711a represent arcs of a circle having a radius of 1 mm.

Further, the example of Figure 7 shows that the sample introduction zone 707 and the sample collection zones 709a, 709b all have a radius of 1.4 mm. The length of the primary fluid path between the sample introduction zone and the sample collection zones 709a, 709b is 10 mm. The width of the primary fluid path is 0.75 mm.

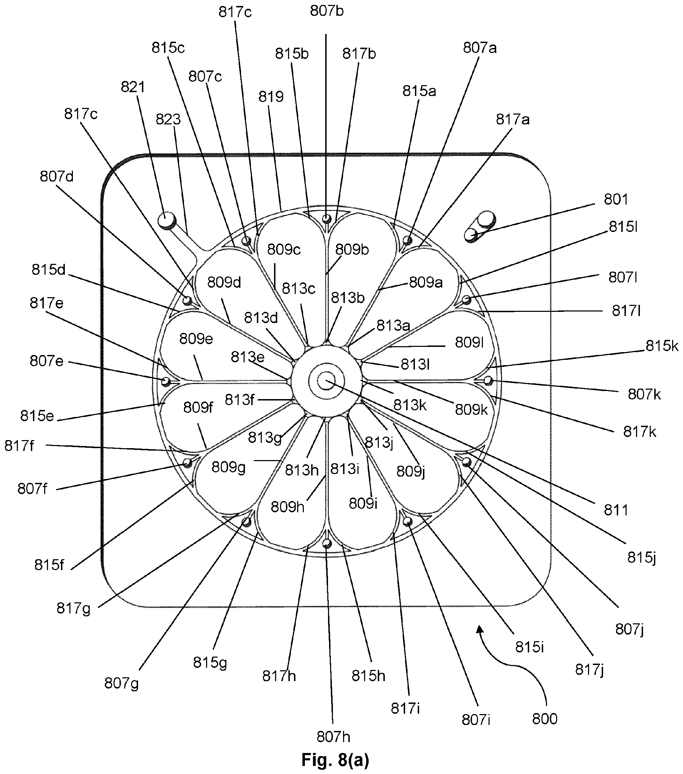

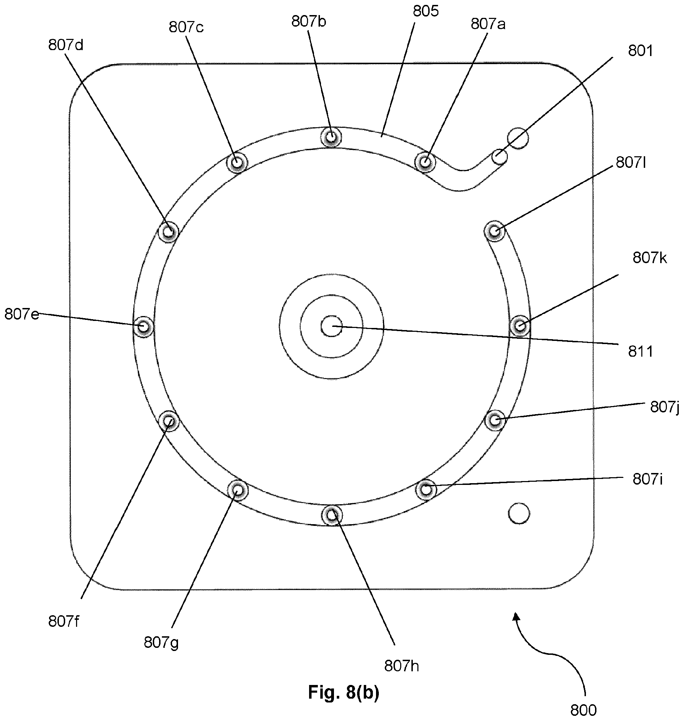

Referring to Figures 8(a) and (b), there is shown another sample separating device. The device 800 is in the form of a microfluidic chip 800.

Figure 8(a) shows a front surface of the device 800. Figure 8(a) shows a fluid inlet 801 where a fluid may be introduced by a fluid delivery unit.

Figure 8(b) shows a rear surface of the device 800. Figure 8(b) shows that fluid entering the fluid inlet 801 from the front surface 800 of the device flows to the rear surface of the device 800 and enters the channel 805. The channel 805 forms a substantial part of a circle. Within the channel are twelve fluid ports 807a - 807I that provide a fluid path between the rear surface of the device 800 and the front surface of the device 800. Fluid flowing through the channel 805 enters the fluid ports 807a-807t and passes to the front surface of the device 800.

Figure 8(a) shows that on the front surface of the device 800, each of the fluid ports 807a-807t is in fluid communication with a microfluidic channel 809a-809l that extends towards the centre of the device 800. A fluid terminus 811 is located at the centre of the device. Thus, it can be seen that fluid flows from the fluid inlet 801 to the fluid terminus 811 via the channel 805, fluid ports 807a-807l, and microchannels 809a-809l. The microchannels 809a - 809l can be considered as providing a plurality of primary fluid paths between the fluid inlet 801 and the fluid terminus 811.

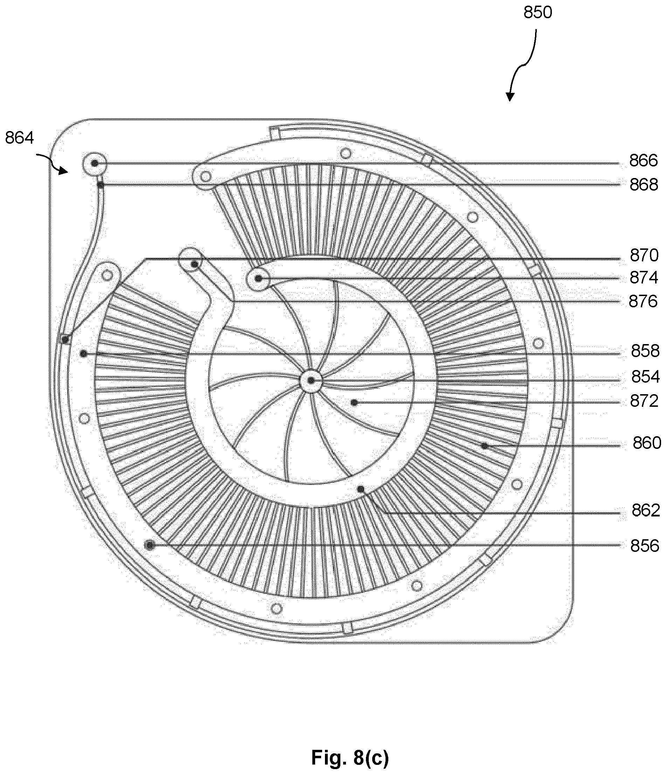

Figure 8(a) further shows that a plurality of sample introduction zones 813a-813l located at the terminal ends of the corresponding microchannels 809a-809l. The sample introduction zones 813a-813l are regions where the sample is introduced into the primary fluid path, such as by pipetting sample into each of the sample introduction zones 813a-813l. Motile organisms in the sample are able to swim against the fluid flow, swim up the microchannels 809a-809t and follow the branching secondary fluid paths 815a,817a - 815l, 817l to a sample collection zone 819. In the sample of Figure 8, a common sample collection zone 819 is provided that is shared with all of the sample introduction zones 813a-813l. That is, each of the sample introduction zones 813a-813l is in fluid communication with the same sample collection zone 819. The sample collection zone 819 is shown in the form of a circular channel 819.

Figure 8(a) further shows a sample extraction point 821 that is in fluid communication with the sample collection zone 819 via channel 823. A pipette may be positioned in the sample extraction point 821 and may be activated to suck up the motile organisms within the sample collection zone 819. The device 800 of Figure 8 may further comprise a cut-off valve (not shown) which may be deployed to cut-off the fluid communication between the sample introduction zones 813a-813l and the sample collection zone 819. The cut-off valve may be beneficial in preventing organisms remaining in the sample introduction zone from being sucked up into a pipette deployed in the sample extraction point 821.

In an example operation of the device of Figures 8(a) and (b), a fluid source such as a syringe may be connected to the fluid inlet 801. The syringe may be activated by a fluid delivery unit such as a syringe pump. Initially, fluid may be delivered into the fluid inlet 801 at a high, device priming flow velocity. The fluid will flow from the fluid inlet 801 into and around the channel 805 on the underside/rear surface of the device 800. As the fluid flows around the channel 805, the fluid will enter the fluid ports 807a-807l, return to the front surface of the device 800 and flow through the microchannels 809a-809l to the fluid terminus 811.

Once the device 800 is primed, the flow velocity of the fluid flow is reduced to the sample introduction flow velocity. Samples are then introduced into the sample introduction zones 813a-813l by one or more pipettes. The sample introduction flow velocity prevents the sample entering the microchannels 809a-809l/sample collection zone 819 by mechanisms such as capillary action or due to the pipetting force.

The flow velocity is then reduced again to the operational flow velocity, and motile organisms in the samples are able to swim against the fluid flow, swim up the microchannels 809a-809t and follow the branching secondary fluid paths 815a,817a - 815l, 817l2 to a sample collection zone 819.

The flow velocity is then increased to a sample collection flow velocity. A pipette is positioned in the sample extraction point 821 and sucks up the motile organisms within the sample collection zone 819. In other words, motile organisms positioned around the sample collection zone 819 are sucked into the sample extraction point 821 by pipetting action. The sample collection flow velocity may be sufficiently high to prevent other organisms remaining in the sample introduction zone 813a-813l from being sucked into the sample collection zone 819 by the pipette. As a further precautionary measure, the cut-off valve (not shown) may be deployed to cut-off the fluid communication between the sample introduction zones 813a-813l and the sample collection zone 819.

Referring to Figure 8(c), there is shown another example sample separating device 850. The device 850 is in the form of a microfluidic chip 850.

Figure 8(c) shows a front surface of the device 850. Figure 8(c) shows fluid inlet 854 where a fluid may be introduced by a fluid delivery unit. The device 850 also comprises a plurality of fluid termini 856 provided in an outlet reservoir 858. As shown in the Figure, the fluid termini 856 are in fluid communication with the fluid inlet 854. A plurality of microfluidic channels 860 extend radially outwardly from a central sample collection zone 862, in the form of arc. Each channel 860 is defined by a pair of channel walls. By providing a plurality of microfluidic channels 860, a greater quantity of sample may be obtaining by the separating method.

The device 850 comprises a sample introduction zone 864 in the form of sample inlet 866 and loading channel 868. The loading channel 868 is an arc surrounding the outlet reservoir 858. The sample introduction zone 864 denotes a region of the device 850 where a sample may be introduced. This is typically performed by pipetting the sample into the sample inlet 866 and allowing it to disperse through the loading channel 868. The sample introduction zone 864 thus allows for uniform distribution of the sample about the microfluidic channels 860. Therefore, there is an increased probability of collecting a high-quality sample by the separating method, as the motile organisms are able to be distributed throughout the loading channel 868 where they can enter the outlet reservoir 858 via a plurality of diffusion channels 870 to access nearby microfluidic channels 860.

The sample collection zone 862 denotes a region of the device 850 where motile organisms may be collected. The sample collection zone 862 is located upstream of the sample introduction zone 864. That is, in use, fluid flows from the sample collection zone 862 to the sample introduction zone 864 downstream, such that motile organisms swim from the sample introduction zone 864 to the sample collection zone 862 upstream. In the example of Figure 8(c), the sample collection zone 862 is provided at the terminal ends of the microchannels 860.

In an example operation of the device of Figure 8(c), a fluid source such as a syringe may be connected to the fluid inlet 854. The syringe may be activated by a fluid delivery unit such as a syringe pump. Initially, during a priming phase, fluid may be delivered into the fluid inlet 854 at a high, device priming flow velocity. The fluid will flow from the fluid inlets 854 into and through the microfluidic channels 860. The fluid exiting the microfluidic channels will then flow to the fluid termini 856.

Notably, fluid flowing from fluid inlet 854 travels into and through a plurality of radially extending flow distribution channels 872. Each flow distribution channel 872 is arcuate, and together the flow distribution channels 872 have a spiral-like form extending outwardly from the fluid inlet 854. Turbulent flow is induced as fluid flows out of the flow distribution channels 872 into the sample collection zone 862. The arcuate shape of the channels 872 can help to facilitate the creation of turbulent flow. In this way, motile organisms that have collected in the sample collection zone 862 are prevented from entering the flow distribution channels 872.

Notably, the fluid flow is uniformly distributed between microfluidic channels 860, such that a substantially identical flow rate is present in each microfluidic channel 860. Furthermore, during priming, fluidic jets are induced in the outlet reservoir at the ends of the microfluidic channels 860 by the high device priming flow velocity. The motile organisms swim toward the jets but are prevented from entering the microfluidic channels 860 by the high flow velocity.

Of course, the flow velocity profiles may be as described above.

Once the device 850 is primed, the flow velocity of the fluid flow is reduced to the sample introduction flow velocity. A sample is then introduced into the sample introduction zone 864. The sample introduction flow velocity prevents the sample entering the microchannels 860 or sample collection zone 862 by mechanisms such as capillary action or due to the pipetting force.

Again, fluidic jets prevent the motile organisms from entering the microchannels 860.

The flow velocity is then reduced again to the operational flow velocity, and motile organisms in the samples are able to swim against the fluid flow, swim up the microchannels 860 to the sample collection zone 862.

Significantly, when the flow rate is reduced to the operational flow velocity, the fluidic jets cease to force the motile organisms away from the microfluidic channels 860, and instead, perhaps in combination with dimensions of the channels or channel openings, induce 3-D counter rotating vortices in the outlet reservoir 858 proximal to the opening of the microfluidic channels 860. These vortices aid transportation of the motile organisms from the sample towards the openings of the microfluidic channels 860. Thus, these vortices encourage motile organisms to the openings of the microfluidic channels 860, These vortices might alternatively or additionally be described as the introduction and maintenance of turbulence proximal to the opening of the microfluidic channels 860.

The flow velocity in the sample collection zone 862 is at a level that motile organisms that enter the sample collection zone 862 remain therein and are not forced back down microchannels 860. Again, as mentioned above, turbulent flows induced in the sample collection zone 862 by fluid flowing from flow distribution channels 872 prevents motile organisms from entering the flow distribution channels 872.

The flow velocity is then increased to a sample collection flow velocity. A sample collection step is then performed. Sample collection can be achieved by use of pipetting action to collect the motile organisms that were able to swim against the fluid flow up the microchannels 860, by pipetting the motile organisms from the sample collection zone 862. The sample collection flow velocity may be sufficiently high to prevent other organisms remaining in the sample introduction zone 864, or motile organisms that were unable to swim completely up the microchannels 860 to the sample collection zone 862, from being sucked into the sample collection zone 862 by the pipette.

Sample collection can alternatively be achieved by performing a flush collection step. The flush collection step makes use of collection zone inlet 874 and collection zone outlet 876. The collection zone inlet 874 and collection zone outlet 876 are in fluid communication with the sample collection zone 862. Fluid may be flushed from the collection inlet 874 to the collection outlet 876, thus removing from the sample collection zone 862 the motile organisms that were able to swim up the microfluidic channels 860 to the sample collection zone 862.

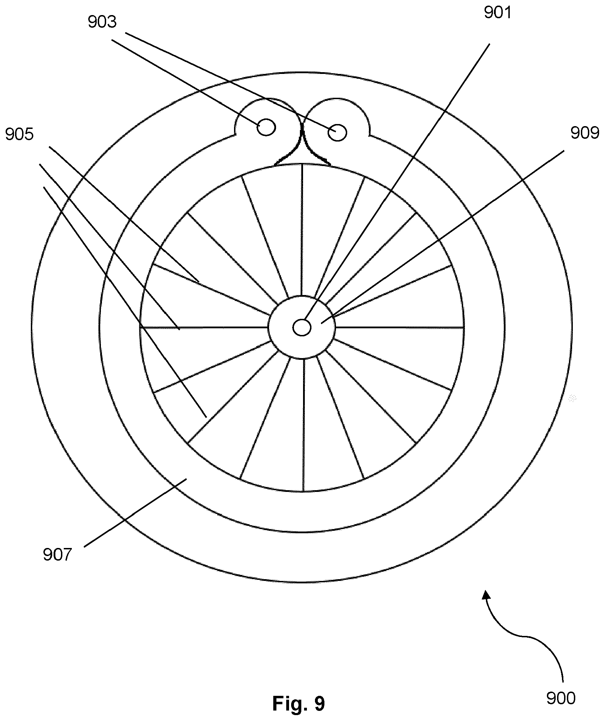

Referring to Figure 9, there is shown another example sample separating device 900. The device 900 comprises a fluid inlet 901. The fluid inlet 901 denotes a point where fluid may enter the device from a fluid delivery unit such as a pump in cooperation with a fluid store. The device 900 also comprises fluid termini 903 in fluid communication with the fluid inlet 901. A plurality of microfluidic channels 905 extend radially outward from a central sample collection zone 909, wherein the fluid inlet 901 is also located. Each channel 905 is defined by a pair of channels walls. By providing a plurality of microfluidic channels 905, a greater quantity of sample may be obtained by the separating method.

The device 900 comprises a sample introduction zone 907 in the form of a ring surrounding the microfluidic channels 905. The sample introduction zone 907 denotes a region of the device 900 where a sample may be introduced. This is typically performed by pipetting the sample into the sample introduction zone 907, typically spread around the ring. The sample introduction zone thus allows for uniform distribution of the sample about the microfluidic channels 905. Therefore, there is an increased probability of collecting a high-quality sample by the separating method, as the motile organisms are able to be distributed throughout the ring where they can suitably access a nearby microfluidic channel 905.

A sample collection zone 909 denotes a region of the device 900 where motile organisms may be collected. The sample collection zone 909 is located upstream of the sample introduction zone 907. That is, in use, fluid flows from the sample collection zone to the sample introduction zone downstream, such that motile organisms swim from the sample introduction zone to the sample collection zone upstream. In the example of Figure 9, the sample collection zone is provided at the terminal ends of microchannels 905.

In an example operation of the device of Figure 9, a fluid source such as a syringe may be connected to the fluid inlet 901. The syringe may be activated by a fluid delivery unit such as a syringe pump. Initially, during a priming phase, fluid may be delivered into the fluid inlet 901 at a high, device priming flow velocity. The fluid will flow from the fluid inlet 901 into and through the microfluidic channels 905. The fluid exiting the microfluidic channels will then flow to the fluid termini 903.

Notably, the fluid flow is uniformly distributed between the channels, such that a substantially identical flow rate is present in each microfluidic channel 905. Furthermore, fluidic jets are caused at the end of the microfluid channels 905 by the high, device priming flow velocity. The motile organisms swim towards the jets but are prevented from entering the microfluidic channels 905. by the high flow velocity.

Of course, the flow velocity profiles may be as described above.

Once the device 900 is primed, the flow velocity of the fluid flow is reduced to the sample introduction flow velocity. A sample is then introduced into the sample introduction zone 907 by one or more pipettes. The sample introduction flow velocity prevents the sample entering the microchannels 905 or sample collection zone 909 by mechanisms such as capillary action or due to the pipetting force.

Again, fluidic jets prevent the motile organisms from entering the channels 905.

The flow velocity is then reduced again to the operational flow velocity, and motile organisms in the samples are able to swim against the fluid flow, swim up the microchannels 905 to the sample collection zone 909.

Significantly, when the flow rate is reduced to the operational flow velocity, the fluidic jets cease to force the motile organisms away from the microfluidic channels 905, and instead, perhaps in combination with dimensions of the channels or channel openings 905, induce 3-D counter-rotating vortices in the sample introduction zone 907 proximal to the opening of the microfluidic channels 905. These vortices aid in the transportation of the motile organisms from the sample towards the opening of the microfluidic channels 905. Thus, these turbulent vortices encourage motile organisms to the opening of the microfluidic channels 905. These vortices might alternatively or additionally be described as the introduction and maintenance of turbulence proximal to the opening of the microfluidic channels 905.

The flow velocity in the sample collection zone 909 is at a level that motile organisms that enter the sample collection one 909 remain therein and are not forced back down microchannels 905.

The flow velocity is then increased to a sample collection flow velocity. Pipetting action is then used to collect the motile organisms that were able to swim against the fluid flow up the microchannels 905, by pipetting the motile organisms from the sample collection zone 909. The sample collection flow velocity may be sufficiently high to prevent other organisms remaining in the sample introduction zone 907, or motile organisms that were unable to swim completely up the microchannels 905 to the sample collection zone 909, from being sucked into the sample collection zone 909 by the pipette.

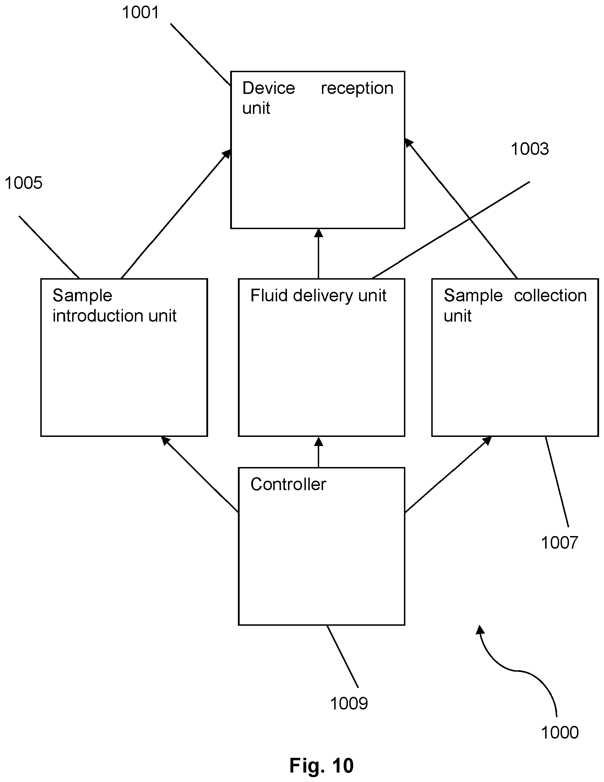

Referring to Figure 10, there is shown an example sample separating apparatus 1000. The sample separating apparatus 1000 is for separating motile organisms from other organisms.

The sample separating apparatus 1000 comprises a device reception unit 1001 arranged to receive a device. The device may be a sample separating device as described above in relation to Figures 5 to 9. The device comprises a fluid inlet, fluid terminus, sample introduction zone, and sample collection zone.

The sample separating apparatus 1000 comprises a fluid delivery unit 1003 in fluid communication with the device reception unit 1001. The fluid delivery unit 1003 is arranged to deliver a fluid to the fluid inlet of the device such that a fluid flow may be established between the fluid inlet and the fluid terminus of the device.

The fluid delivery unit may comprise a fluid source and a pump. The pump may operate by suction or by pressure. The pump may be a microfluidic pump. The pump may be a syringe pump.

The sample separating apparatus 1000 comprises a sample introduction unit 1005 arranged to introduce a sample to the sample introduction zone of the device such that motile organisms introduced into the sample introduction zone are able to swim against the fluid flow and enter the sample collection zone.

The sample separating apparatus 1000 further comprises a sample collection unit 1007 arranged to collect the sample from the sample collection zone of the device.

The sample separating apparatus 1000 further comprises a controller 1009. The controller 1009 is operable to control the fluid delivery unit 1001, the sample introduction unit 1005, and the sample collection unit 1007. The controller 1009 enables the sample separating apparatus 1000 to perform certain actions automatically and without a user input.

In operation, a sample separating device is positioned in the device reception unit 1001. Positioning the device in the device reception unit 1001 connects the fluid inlet of the device to the fluid delivery unit 1003 such that a fluid flow may be established between the fluid inlet and the fluid terminus of the device. The fluid delivery unit 1003 may be controlled by the controller 1009 to deliver fluid at different flow velocities such as those described above in relation to the first aspect of the invention. The controller 1009 controls the sample introduction unit 1005 to introduce a sample into the sample introduction zone. Once the sample separating operation has finished, the controller 1009 controls the sample collection unit 1007 to collect the sample from the sample collection zone. All or part of this operation may be performed automatically without user input.

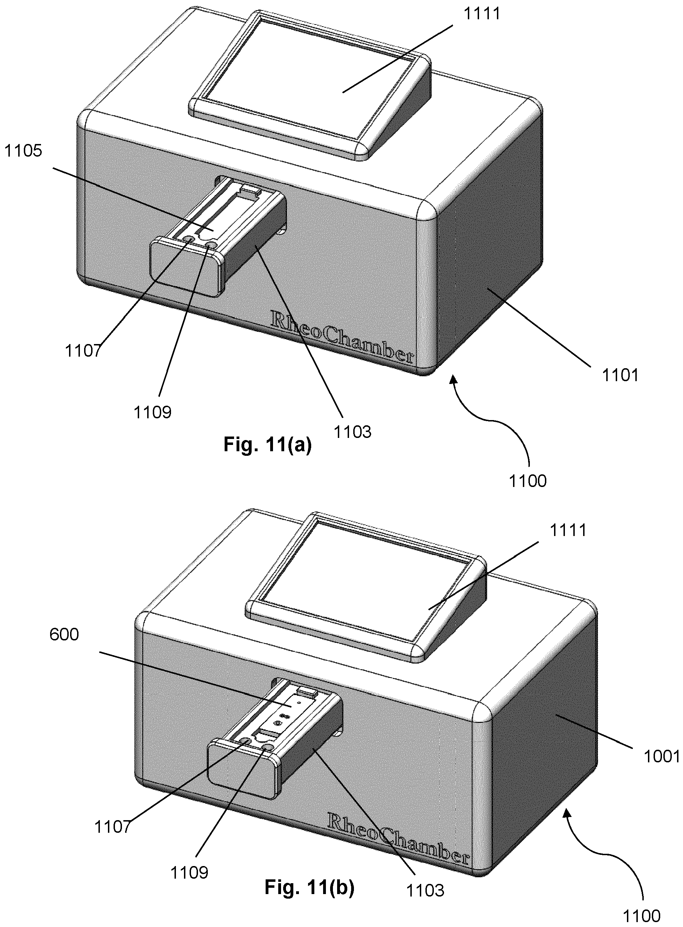

Referring to Figures 11(a) and 11(b) there is shown a sample separating apparatus 1100. The sample separating apparatus 1100 comprises a housing 1101. The sample separating apparatus 1100 comprises a device reception unit 1103 in the form of a moveable tray 1103. The moveable tray 1103 comprises a recess 1105 for receiving a device, a recess 1107 for receiving a sample tube, and a recess 1109 for receiving a collection tube. The sample tube holds a sample for introduction to the sample introduction zone by the sample introduction unit of the apparatus 1100. The collection tube is for receiving a sample collected from the sample collection zone by the sample collection unit of the apparatus 1100. The moveable tray 1103 is shown in Figures 11(a) and 11(b) in an open configuration. In this configuration a device, sample tube, and collection tube may be positioned/removed from the moveable tray 1103. Figure 11(b) shows a device 600 loaded into the recess 1105 in the moveable tray 1103. The moveable tray 1103 is able to be pushed into a closed configuration where the tray 1103 is enclosed within the housing 1101. This has the effect of enclosing a device loaded into the tray 1103 into the housing 1101. This helps to protect the device from environmental disturbances during the sample separating operation.

After the tray 1103, the device is sealed from the external environment and thus is protected from local atmospheric pressure changes within the environment where the apparatus 1100 is located. A temperature control unit of the apparatus is further operable to heat the vicinity of the device to a predetermined temperature value and maintain this the temperature at this value. The predetermined temperature value may be body temperature (e.g. 37 degrees centigrade).

Once the temperature has stabilised, the method as described above in relation to the first aspect of the present invention may be performed. That is, the device is primed with fluid by the fluid delivery unit delivering fluid at the device priming flow velocity. The fluid delivery unit is controlled to reduce the fluid flow velocity to the sample introduction flow velocity, and the sample introduction unit operates to introduce a sample into the sample introduction zone of the device. The sample introduction flow velocity is then gradually reduced to an operational flow velocity, and the fluid flow is maintained at the operational flow velocity until sufficient motile organisms have swum against the fluid flow and entered the sample collection zone. The fluid delivery unit is then controlled to increase the fluid flow to the sample collection flow velocity, and the sample collection unit is activated to collect the sample from the sample collection zone.

In the example of Figures 11(a)-(b), the apparatus 1100 further comprises a display 1111. The display 1111 provides a user interface through which a user may interact to control the apparatus 1100. Here, control the apparatus 1100 may mean control the overall operation of the apparatus 1100. Individual steps or operations may be performed by the apparatus 1100 automatically without a specific user input. The apparatus 1100 may further comprise an imaging unit operable to image a device received in the device reception unit. The display 1111 is operable to display the imaged device. This is useful as it enables a user to visually inspect the device, and in particular, the sample collection zone so that a user can confirm whether a sufficient number of motile organisms have entered the sample collection zone.

Beneficially, the sample separating method, device, and apparatus disclosed herein are able to separate motile organisms, such as motile sperm, from other organisms within an original sample. In experiments performed on frozen and fresh sperm samples, approaches in accordance with the present invention provide samples in the sample collection zones of the device with a higher percentage concentration of motile sperm compared to existing approaches. Moreover, a greater percentage of the sperm within the sample collection zone were found to have a normal morphology than in existing approaches. Beneficially still, the sperm within the sample collection zone were found to have a lower DNA Fragmentation Index than in existing approaches.

In experiments performed on mice using ICSI, the cleavage rate (i.e. the fertilisation rate) was found to be higher for sperm separated according to approaches of the present invention than in existing approaches. Moreover, the Morula-Blastocyst Rate (an indication of the embryo development rate) was found to be higher for sperm separated according to approaches of the present invention than in existing approaches. The implantation rate and the foetus development rate were also found to be higher when sperm separated according to approaches of the present invention were used. In addition, the reabsorption rate for sperm separated according to approaches of the present invention were lower.

Individually, the sample separating method, the sample separating device, and the sample separating apparatus are able to provide benefits in terms of separating motile organisms from other organisms within a sample. Significantly still, synergistic benefits can be achieved through a combination of the sample separating method and the sample separating device, the sample separating method and the sample separating apparatus, the sample separating device and the sample separating apparatus, or the sample separating method, device, and apparatus.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. The described elements may be configured to reside on a tangible, persistent, addressable storage medium and may be configured to execute on one or more processors. These functional elements may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables.

## Claims

1. A sample separating method for separating motile organisms from other organisms, the method comprising:
controlling a fluid delivery unit to provide a fluid flow to a sample separating device comprising a fluid inlet and a fluid terminus, wherein the fluid flow is provided between the fluid inlet and the fluid terminus of the device, wherein the fluid flow has a sample introduction flow velocity set so that a sample may be introduced into a sample introduction zone of the device, and wherein the sample introduction flow velocity is sufficiently high such that an organism in the sample is unable to exit the sample introduction zone; and
controlling the fluid delivery unit to reduce the fluid flow velocity from the sample introduction flow velocity to an operational flow velocity lower than the sample introduction flow velocity, wherein the operational flow velocity is selected such that motile organisms in the sample are able to swim against the fluid flow and enter a sample collection zone of the device,
**characterised in that**
controlling the fluid delivery unit to set the fluid flow to have the operational flow velocity comprises reducing the fluid flow velocity from the sample introduction flow velocity to the operational flow velocity over a predetermined period of time.

2. A method as claimed in claim 1, wherein the predetermined period of time is greater than or equal to 30 seconds, optionally greater than or equal to 1 minute, optionally between 1 minute and 60 minutes, optionally between 1 minute and 20 minutes, and optionally between 1 minute and 10 minutes.

3. A method as claimed in any preceding claim, wherein the sample introduction flow velocity is greater than or equal to 100 micrometres per second, optionally between 100 micrometres per second to 1000 micrometres per second, optionally between 200 micrometres per second to 800 micrometres per second, for example 550 micrometres per second.

4. A method as claimed in any preceding claim, wherein the operational flow velocity is greater than or equal to 20 micrometres per second, optionally between 20 micrometres per second to 150 micrometres per second, and optionally between 30 micrometres per second to 80 micrometres per second, for example 55 micrometres per second.

5. A method as claimed in any preceding claim, wherein controlling the fluid delivery unit to set the fluid flow to the operational flow velocity comprises maintaining the fluid flow substantially at the operational flow velocity over a predetermined period of time, optionally wherein the predetermined period of time is greater than or equal to 1 minute, optionally between 1 minute and 120 minutes, and optionally between 10 minutes to 45 minutes, for example 30 minutes.

6. A method as claimed in any preceding claim, further comprising controlling the fluid delivery unit to increase the fluid flow velocity from the operational flow velocity to a sample collection flow velocity higher than the operational flow velocity such that motile organisms are able to be collected from the sample collection zone, optionally wherein the sample collection flow velocity is greater than or equal to 150 micrometres per second, optionally between 150 micrometres per second and 15000 micrometres per second, for example 250 micrometres per second, and optionally between 400 micrometres per second and 11000 micrometres per second, for example 11000 micrometres per second.

7. A method as claimed in any preceding claim, wherein prior to controlling the fluid delivery unit to set the fluid flow to have the sample introduction flow velocity, the method comprises controlling the fluid flow to have a device priming flow velocity, wherein the device priming flow velocity is higher than the sample introduction flow velocity, optionally wherein the device priming flow velocity is greater than or equal to 800 micrometres per second, optionally between 800 and 2000 micrometres per second, and optionally between 1500 and 1600 micrometres per second, for example 1100 micrometres per second.

8. A method as claimed in any preceding claim, wherein the motile organisms are organisms that exhibit a form of taxis, optionally wherein the motile organisms are sperm.

9. A computer readable medium having instructions recorded thereon which, when executed by a computer, cause the computer to perform the method by controlling the fluid delivery unit as claimed in any of claims 1 to 8.

10. A sample separating apparatus arranged to provide a fluid flow to a device for separating motile organisms from other organisms, the device comprises a fluid inlet, a fluid terminus, a sample introduction zone and a sample collection zone, the apparatus comprises a fluid delivery unit; and a controller operable to control the fluid delivery unit to perform the method as claimed in any of claims 1 to 8.

11. A sample separating apparatus as claimed in claim 10 comprising one or more channels, each channel being defined by a pair of channel walls between the sample introduction zone and the sample collection zone.

12. A sample separating apparatus as claimed in claim 11 comprising a plurality of channels,
optionally wherein each channel extends radially outward from a central sample collection zone to the sample introduction zone,
optionally wherein the sample introduction zone extends around the collection zone.

## Patentansprüche

1. Verfahren zur Probentrennung zum Trennen beweglicher Organismen von anderen Organismen, das Verfahren umfassend:
Steuern einer Fluidabgabeeinheit, um einen Fluidstrom zu einer Probentrennvorrichtung bereitzustellen, umfassend einen Fluideinlass und einen Fluidabschluss, wobei der Fluidstrom zwischen dem Fluideinlass und dem Fluidabschluss der Vorrichtung bereitgestellt wird, wobei der Fluidstrom eine Probeneinführungsströmungsgeschwindigkeit aufweist, die so eingestellt ist, dass eine Probe in eine Probeneinführungszone der Vorrichtung eingeführt werden kann, und wobei die Probeneinführungsströmungsgeschwindigkeit ausreichend hoch ist, sodass ein Organismus in der Probe nicht in der Lage ist, die Probeneinführungszone zu verlassen; und
Steuern der Fluidabgabeeinheit, um die Fluidströmungsgeschwindigeit von der Probeneinführungsströmungsgeschwindigkeit auf eine Betriebsströmungsgeschwindigkeit zu verringern, die niedriger ist als die Probeneinführungsströmungsgeschwindigkeit, wobei die Betriebsströmungsgeschwindigkeit derart ausgewählt ist, dass bewegliche Organismen in der Probe in der Lage sind, gegen die Fluidströmung zu schwimmen und in eine Probensammelzone der Vorrichtung einzutreten,
**dadurch gekennzeichnet, dass**
das Steuern der Fluidabgabeeinheit, um die Fluidströmung auf die Betriebsströmungsgeschwindigkeit einzustellen, umfasst, dass die Fluidströmungsgeschwindigeit von der Probeneinführungsströmungsgeschwindigkeit über einen vorgegebenen Zeitraum auf die Betriebsströmungsgeschwindigkeit verringert wird.

2. Verfahren nach Anspruch 1, wobei der vorgegebene Zeitraum mindestens 30 Sekunden beträgt, optional mindestens 1 Minute, optional zwischen 1 Minute und 60 Minuten, optional zwischen 1 Minute und 20 Minuten und optional zwischen 1 Minute und 10 Minuten.

3. Verfahren nach einem der vorhergehenden Ansprüche, wobei die Probeneinführungsströmungsgeschwindigkeit mindestens 100 Mikrometer pro Sekunde beträgt, optional zwischen 100 Mikrometer pro Sekunde und 1000 Mikrometer pro Sekunde, optional zwischen 200 Mikrometer pro Sekunde und 800 Mikrometer pro Sekunde, wie zum Beispiel 550 Mikrometer pro Sekunde.

4. Verfahren nach einem der vorhergehenden Ansprüche, wobei die Betriebsströmungsgeschwindigkeit mindestens 20 Mikrometer pro Sekunde beträgt, optional zwischen 20 Mikrometer pro Sekunde und 150 Mikrometer pro Sekunde und optional zwischen 30 Mikrometer pro Sekunde und 80 Mikrometer pro Sekunde, wie zum Beispiel 55 Mikrometer pro Sekunde.

5. Verfahren nach einem der vorhergehenden Ansprüche, wobei das Steuern der Fluidabgabeeinheit, um die Fluidströmung auf die Betriebsströmungsgeschwindigkeit einzustellen, das Halten der Fluidströmung über einen vorgegebenen Zeitraum im Wesentlichen auf der Betriebsströmungsgeschwindigkeit umfasst, optional wobei der vorgegebene Zeitraum mindestens 1 Minute beträgt, optional zwischen 1 Minute und 120 Minuten und optional zwischen 10 Minuten und 45 Minuten, wie zum Beispiel 30 Minuten.

6. Verfahren nach einem der vorhergehenden Ansprüche, ferner umfassend das Steuern der Fluidabgabeeinheit, um die Fluidströmungsgeschwindigeit von der Betriebsströmungsgeschwindigkeit auf eine Probensammelströmungsgeschwindigkeit zu erhöhen, die höher ist als die Betriebsströmungsgeschwindigkeit, sodass bewegliche Organismen aus der Probensammelzone gesammelt werden können, optional wobei die Probensammelströmungsgeschwindigkeit mindestens 150 Mikrometer pro Sekunde beträgt, optional zwischen 150 Mikrometer pro Sekunde und 15000 Mikrometer pro Sekunde, zum Beispiel 250 Mikrometer pro Sekunde, und optional zwischen 400 Mikrometer pro Sekunde und 11000 Mikrometer pro Sekunde, zum Beispiel 11000 Mikrometer pro Sekunde.

7. Verfahren nach einem der vorhergenden Ansprüche, wobei vor dem Steuern der Fluidabgabeeinheit so, dass die Fluidströmung die Probeneinführungsströmungsgeschwindigkeit aufweist, das Verfahren das Steuern der Fluidströmung derart umfasst, das sie eine Vorrichtungsansauggeschwindigkeit aufweist, wobei die Vorrichtungsansauggeschwindigkeit höher ist als die Probeneinführungsströmungsgeschwindigkeit, optional wobei die Vorrichtungsansauggeschwindigkeit mindestens 800 Mikrometer pro Sekunde beträgt, optional zwischen 800 und 2000 Mikrometer pro Sekunde und optional zwischen 1500 und 1600 Mikrometer pro Sekunde, zum Beispiel 1100 Mikrometer pro Sekunde.

8. Verfahren nach einem der vorhergehenden Ansprüche, wobei bewegliche Organismen Organismen sind, die eine Form von Taxis aufweisen, optional wobei die beweglichen Organismen Spermien sind.

9. Computerlesbares Medium mit darauf aufgezeichneten Anweisungen, die, wenn durch einen Computer ausgeführt, bewirken, dass der Computer das Verfahren durch Steuern der Fluidabgabeeinheit nach einem der Ansprüche 1 bis 8 ausführt.

10. Probentrenneinrichtung, angeordnet zum Bereitstellen einer Fluidströmung an eine Vorrichtung zum Trennen von beweglichen Organismen von anderen Organismen, wobei die Vorrichtung einen Fluideinlass, einen Fluidabschluss und eine Probensammelzone umfasst, wobei die Vorrichtung eine Fluidabgabeeinheit umfasst; und eine Steuerung, betreibbar zum Steuern der Fluidabgabeeinheit, um das Verfahren nach einem der Ansprüche 1 bis 8 auszuführen.

11. Probentrenneinrichtung nach Anspruch 10, umfassend einen oder mehrere Kanäle, wobei jeder Kanal durch ein Paar von Kanalwänden zwischen der Probeneinführungszone und der Probensammelzone definiert ist.

12. Probentrenneinrichtung nach Anspruch 11, umfassend eine Vielzahl von Kanälen,
optional wobei jeder Kanal sich von einer mittigen Probensammelzone radial hin zu der Probeneinführungszone erstreckt,
optional wobei sich die Probeneinführungszone um die Probensammelzone herum erstreckt.

## Revendications

1. Procédé de séparation d'échantillons pour séparer des organismes motiles d'autres organismes, le procédé comprenant :
la commande d'une unité de distribution de fluide pour fournir un écoulement de fluide à un dispositif de séparation d'échantillons comprenant une entrée de fluide et un terminus de fluide, l'écoulement de fluide étant fourni entre l'entrée de fluide et le terminus de fluide du dispositif, l'écoulement de fluide ayant un ensemble de vitesse d'écoulement d'introduction d'échantillon de sorte qu'un échantillon puisse être introduit jusque dans une zone d'introduction d'échantillon du dispositif, et la vitesse d'écoulement d'introduction d'échantillon étant suffisamment élevée de telle sorte qu'un organisme dans l'échantillon ne puisse pas sortir de la zone d'introduction d'échantillon ; et
la commande de l'unité de distribution de fluide pour réduire la vitesse d'écoulement de fluide de la vitesse d'écoulement d'introduction d'échantillon à une vitesse d'écoulement opérationnelle inférieure à la vitesse d'écoulement d'introduction d'échantillon, la vitesse d'écoulement opérationnelle étant sélectionnée de telle sorte que des organismes motiles dans l'échantillon puissent nager contre l'écoulement de fluide et entrer dans une zone de collecte d'échantillon du dispositif,
**caractérisé en ce que**
la commande de l'unité de distribution de fluide pour régler l'écoulement de fluide afin d'avoir la vitesse d'écoulement opérationnelle comprend la réduction de la vitesse d'écoulement de fluide de la vitesse d'écoulement d'introduction d'échantillon à la vitesse d'écoulement opérationnelle sur une période de temps prédéterminée.

2. Procédé selon la revendication 1, dans lequel la période prédéterminée est supérieure ou égale à 30 secondes, facultativement supérieure ou égale à 1 minute, facultativement comprise entre 1 minute et 60 minutes, facultativement comprise entre 1 minute et 20 minutes, et facultativement comprise entre 1 minute et 10 minutes.

3. Procédé selon l'une quelconque des revendications précédentes, dans lequel la vitesse d'écoulement d'introduction d'échantillon est supérieure ou égale à 100 micromètres par seconde, facultativement comprise entre 100 micromètres par seconde et 1 000 micromètres par seconde, facultativement comprise entre 200 micromètres par seconde et 800 micromètres par seconde, par exemple 550 micromètres par seconde.

4. Procédé selon l'une quelconque des revendications précédentes, dans lequel la vitesse d'écoulement opérationnelle est supérieure ou égale à 20 micromètres par seconde, facultativement comprise entre 20 micromètres par seconde et 150 micromètres par seconde, et facultativement comprise entre 30 micromètres par seconde et 80 micromètres par seconde, par exemple 55 micromètres par seconde.

5. Procédé selon l'une quelconque des revendications précédentes, dans lequel la commande de l'unité de distribution de fluide pour régler l'écoulement de fluide à la vitesse d'écoulement opérationnelle comprend le maintien de l'écoulement de fluide substantiellement à la vitesse d'écoulement opérationnelle sur une période de temps prédéterminée, facultativement dans lequel la période de temps prédéterminée est supérieure ou égale à 1 minute, facultativement comprise entre 1 minute et 120 minutes, et facultativement comprise entre 10 minutes et 45 minutes, par exemple 30 minutes.

6. Procédé selon l'une quelconque des revendications précédentes, comprenant en outre la commande de l'unité de distribution de fluide pour augmenter la vitesse d'écoulement de fluide de la vitesse d'écoulement opérationnelle à une vitesse d'écoulement de collecte d'échantillon supérieure à la vitesse d'écoulement opérationnelle de telle sorte que des organismes motiles puissent être collectés à partir de la zone de collecte d'échantillon, facultativement la vitesse d'écoulement de collecte d'échantillon étant supérieure ou égale à 150 micromètres par seconde, facultativement comprise entre 150 micromètres par seconde et 15 000 micromètres par seconde, par exemple 250 micromètres par seconde, et facultativement comprise entre 400 micromètres par seconde et 11 000 micromètres par seconde, par exemple 11 000 micromètres par seconde.

7. Procédé selon l'une quelconque des revendications précédentes, dans lequel avant la commande de l'unité de distribution de fluide pour régler l'écoulement de fluide afin d'avoir la vitesse d'écoulement d'introduction d'échantillon, le procédé comprend la commande de l'écoulement de fluide pour avoir une vitesse d'écoulement d'amorçage de dispositif, la vitesse d'écoulement d'amorçage de dispositif étant supérieure à la vitesse d'écoulement d'introduction d'échantillon, facultativement la vitesse d'écoulement d'amorçage de dispositif étant supérieure ou égale à 800 micromètres par seconde, facultativement comprise entre 800 et 2 000 micromètres par seconde, et facultativement comprise entre 1 500 et 1 600 micromètres par seconde, par exemple 1 100 micromètres par seconde.

8. Procédé selon l'une quelconque des revendications précédentes, dans lequel les organismes motiles sont des organismes qui présentent une forme de taxis, facultativement les organismes motiles étant du sperme.

9. Support lisible par ordinateur sur lequel sont enregistrées des instructions qui, lorsqu'elles sont exécutées par un ordinateur, amènent l'ordinateur à réaliser le procédé en commandant l'unité de distribution de fluide selon l'une quelconque des revendications 1 à 8.

10. Appareil de séparation d'échantillons agencé pour fournir un écoulement de fluide à un dispositif afin de séparer des organismes motiles d'autres organismes, le dispositif comprend une entrée de fluide, un terminus de fluide, une zone d'introduction d'échantillon et une zone de collecte d'échantillon, l'appareil comprend une unité de distribution de fluide ; et un dispositif de commande pouvant être opérationnel pour commander l'unité de distribution de fluide afin de réaliser le procédé selon l'une quelconque des revendications 1 à 8.

11. Appareil de séparation d'échantillons selon la revendication 10 comprenant un ou plusieurs canaux, chaque canal étant défini par une paire de parois de canal entre la zone d'introduction d'échantillon et la zone de collecte d'échantillon.

12. Appareil de séparation d'échantillons selon la revendication 11, comprenant une pluralité de canaux,
facultativement dans lequel chaque canal s'étend radialement vers l'extérieur à partir d'une zone centrale de collecte d'échantillon jusqu'à la zone d'introduction d'échantillon,
facultativement dans lequel la zone d'introduction d'échantillon s'étend autour de la zone de collecte.
